# FASCICULE DE BREVET EUROPEEN

(11) **EP 2 370 446 B1**
(45) Date de publication et mention de la délivrance du brevet: **25.11.2015**
(21) Numéro de dépôt: 09797121.2
(22) Date de dépôt: 30.11.2009
(51) Int. Cl.: C07D 519/00

(54) **DÉRIVÉS DE 6-CYCLOAMINO-3-(1H-PYRROLO[2,3-b]PYRIDIN-4-YL)IMIDAZO[1,2-b]-PYRIDAZINE, LEUR PRÉPARATION ET LEUR APPLICATION EN THÉRAPEUTIQUE**
6-CYCLOAMINO-3-(1H-PYRROLO[2,3-B]PYRIDIN-4-YL)IMIDAZO[1,2-B]PYRIDAZINDERIVATE, DEREN HERSTELLUNG UND DEREN THERAPEUTISCHE VERWENDUNG
6-CYCLOAMINO-3-(1H-PYRROLO[2,3-B]PYRIDIN-4-YL)IMIDAZO[1,2-B]PYRIDAZINE DERIVATIVES,PREPARATION THEREOF AND THERAPEUTIC USE THEREOF

(30) Priorité: 01.12.2008 FR 0806723
(43) Date de publication de la demande: 05.10.2011
(73) Titulaire: SANOFI, 75008 Paris (FR)
(72) Inventeur: PACAUD, Christophe, F-75013 Paris (FR); PUECH, Frédéric, F-PARIS 75013 (FR)
(74) Mandataire: Le Coupanec, Pascale A.M.P.
(86) Numéro de dépôt international: PCT/FR2009/052336
(87) Numéro de publication internationale: WO 2010/063929

(56) Documents cités:
- WO-A-2007/025540
- WO-A-2008/138834
- WO-A-2008/138889

## Description

La présente invention se rapporte à des dérivés de 6-cycloamino-3-(1*H*-pyrrolo[2,3-*b*]pyridin-4-yl)imidazo[1,2-b]pyridazine, à leur préparation et à leur application en thérapeutique, dans le traitement ou la prévention de maladies impliquant la caséine kinase 1 epsilon et/ou la caséine kinase 1 delta.

La présente invention a pour objet les composés répondant à la formule générale (I) 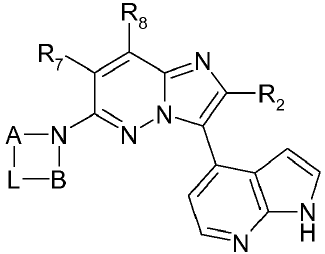 dans laquelle :
- R₂ représente un groupe aryle éventuellement substitué par un ou plusieurs substituants choisis parmi les atomes d'halogène et les groupes C₁₋₆-alkyle, C₁₋₆-alkyloxy, C₁₋₆-alkylthio, C₁₋₆-fluoroalkyle, C₁₋₆-fluoroalkyloxy, -CN ou R₂ représente un groupe C₁₋₆-alkyle, C₁₋₆-fluoroalkyle, C₃₋₇-cycloalkyle, C₃₋₇-cycloalkyl-C₁₋₆-alkyle ;
- A représente un groupe C₁₋₇-alkylène éventuellement substitué par un ou deux groupes Rₐ;
- B représente un groupe C₁₋₇-alkylène éventuellement substitué par un groupe R_{b} ;
- L représente, soit un atome d'azote éventuellement substitué par un groupe R_{c} ou R_{d}, soit un atome de carbone substitué par un groupe Rₑ₁ et un groupe R_{d} ou deux groupes Rₑ₂ ;
les atomes de carbone de A et de B étant éventuellement substitués par un ou plusieurs groupes R_{f} identiques ou différents l'un de l'autre ;
- Rₐ, R_{b} et R_{c} sont définis tels que :
   deux groupes Rₐ peuvent former ensemble un groupe C₁₋₆-alkylène ;
   Rₐ et R_{b} peuvent former ensemble une liaison ou un groupe C₁₋₆-alkylène ;
   Rₐ et R_{c} peuvent former ensemble une liaison ou un groupe C₁₋₆-alkylène ;
   R_{b} et R_{c} peuvent former ensemble une liaison ou un groupe C₁₋₆-alkylène ;
- R_{d} représente un groupe choisi parmi l'atome d'hydrogène et les groupes C₁₋₆-alkyle, C₃₋₇-cycloalkyle, C₃₋₇-cycloalkyl-C₁₋₆-alkyle, hydroxy-C₁₋₆-alkyle, C₁₋₆-alkyloxy-C₁₋₆-alkyle, C₁₋₆-alkylthio-C₁₋₆-alkyle, C₁₋₆-fluoroalkyle ou benzyle ;
- Rₑ₁ représente un groupe -NR₄R₅ ou une monoamine cyclique comportant éventuellement un atome d'oxygène, la monoamine cyclique étant éventuellement substituée par un ou plusieurs substituants choisis parmi l'atome de fluor et les groupes C₁₋₆-alkyle, C₁₋₆-alkyloxy, hydroxyle ;
- Deux Rₑ₂ forment avec l'atome de carbone qui les porte une monoamine cyclique comportant éventuellement un atome d'oxygène, la monoamine cyclique étant éventuellement substituée par un ou plusieurs groupes R_{f} identiques ou différents l'un de l'autre ;
- R_{f} représente un groupe C₁₋₆-alkyle, C₃₋₇-cycloalkyle, C₃₋₇-cycloalkyle-C₁₋₆-alkyle, C₁₋₆-alkyloxy-C₁₋₆-alkyle, hydroxy-C₁₋₆-alkyle, C₁₋₆-fluoroalkyle, phényle ou benzyle ;
- R₄ et R₅ représentent, indépendamment l'un de l'autre, un atome d'hydrogène ou un groupe C₁₋₆-alkyle, C₃₋₇-cycloalkyle, C₃₋₇-cycloalkyle-C₁₋₆-alkyle ;
- R₇ et R₈ représentent, indépendamment l'un de l'autre, un atome d'hydrogène ou un groupe C₁₋₆-alkyle.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention. Ces sels sont avantageusement préparés avec des acides pharmaceutiquement acceptables, mais les sels d'autres acides utiles, par exemple, pour la purification ou l'isolement des composés de formule (J) font également partie de l'invention.

On connait de l'art antérieur, le document WO 2008/138834 qui divulgue des dérivés d'imidazo[1,2-b]pyridazines 2,3,6-trisubstitués de formule générale (1) suivante en tant qu'inhibiteurs de kinases de la famille des protéines kinases liées à la kinase PI3 :

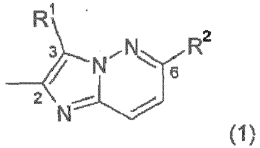

Toutefois, les composés de formule (1) ne possèdent pas d'azacycle en guise de groupe R².

On connait également de l'art antérieur, Je document WO 2008/138889 qui décrit des dérivés d'imidazo[1,2-b]pyridazines 3,6-disubstitués de formule générale (2) suivante en tant qu'inhibiteurs de kinases de la famille des protéines kinases liées à la kinase PI3 :

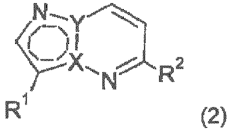

Toutefois, ces composés ne sont pas substitués en position 2.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Dans le cadre de l'invention, on entend par :
- C_{t-z} où t et z peuvent prendre les valeurs de 1 à 7, une chaîne carbonée pouvant avoir de t à z atomes de carbone, par exemple C₁₋₇ une chaîne carbonée qui peut avoir de 1 à 7 atomes de carbone
- alkyle, un groupe aliphatique saturé, linéaire ou ramifié ; par exemple un groupe C₁₋₇-alkyle représente une chaîne carbonée de 1 à 7 atomes de carbone, linéaire ou ramifiée, par exemple un méthyle, éthyle, propyle, isopropyle, butyle, isobutyle, terbutyle, pentyle, hexyle, heptyle ;
- alkylène, un groupe alkyle divalent saturé, linéaire ou ramifié, par exemple un groupe C₁₋₆-alkylène représente une chaîne carbonée divalente de 1 à 6 atomes de carbone, linéaire ou ramifiée, par exemple un méthylène, éthylène, 1-méthyléthylène, propylène, butylène ;
- cycloalkyle, un groupe alkyle cyclique, par exemple un groupe C₃₋₇-cycloalkyle représente un groupe carboné cyclique de 3 à 7 atomes de carbone, par exemple un cyclopropyle, cyclobutyle, cyclopentyle, cyclohexyle, cycloheptyle
- hydroxyle, un groupe -OH ;
- -CN, un groupe nitrile ;
- monoamine cyclique, une chaîne carbonée saturée cyclique ou polycyclique, éventuellement pontée ou fusionnée comportant 1 atome d'azote ;

A titre d'exemple de monoamine cyclique formée par N, A, L et B comportant éventuellement un atome d'oxygène, on peut notamment citer l'aziridine, l'azétidine, la pyrrolidine, la pipéridine, l'azépine, la morpholine, l'homopipéridine, la décahydroquinoline, la décahydroisoquinoline, l'azabicyclo-heptane, l'azabicyclo-octane, l'azabicyclo-nonane, l'aza-oxo-bicyclo-heptane, l'aza-oxo-bicyclo-octane ;
- hydroxyakyle, un groupe alkyle dont un atome d'hydrogène a été substitué par un groupe hydroxyle ;
- alkyloxy, un groupe -O-alkyle ;
- alkylthio, un groupe -S-alkyle ;
- fluoroalkyle, un groupe alkyle dont un ou plusieurs atomes d'hydrogène ont été substitués par un atome de fluor ;
- fluoroalkyloxy, un groupe alkyloxy dont un ou plusieurs atomes d'hydrogène ont été substitués par un atome de fluor ;
- un atome d'halogène, un atome de fluor, de chlore, de brome ou d'iode ;
- aryle, un groupe aromatique mono- ou bicyclique comprenant entre 6 et 10 atomes de carbone. A titre d'exemple de groupe aryle, on peut citer les groupes phényle ou naphtyle.

Parmi les composés de formule générale (I) objets de l'invention, un premier groupe de composés est constitué par les composés pour lesquels R₂ représente un phényle, éventuellement substitué par un ou plusieurs atomes d'halogène ou groupes C₁₋₆-alkyle, C₁₋₆-fluoroalkyle ;
A, L, B, R₇ et R₈ étant tels que définis ci-dessus.

Parmi les composés de formule générale (I) objets de l'invention, un deuxième groupe de composés est constitué par les composés pour lesquels R₂ représente un phényle éventuellement substitué par un ou plusieurs atomes de fluor ;
A, L, B, R₇ et R₈ étant tels que définis ci-dessus.

Parmi les composés de formule générale (I) objets de l'invention, un troisième groupe de composés est constitué par les composés pour lesquels R₂ représente un 3-fluoro-phényle ou un 4-fluoro-phényle ;
A, L, B, R₇ et R₈ étant tels que définis ci-dessus.

Parmi les composés de formule générale (I) objets de l'invention, un quatrième groupe de composés est constitué par les composés pour lesquels R₂ représente un groupe C₁₋₆-alkyle, C₁₋₆-fluoroalkyle, C₃₋₇-cycloalkyle, C₃₋₇-cycloalkyl-C₁₋₆-alkyle;
A, L, B, R₇ et R₈ étant tels que définis ci-dessus.

Parmi les composés de formule générale (I) objets de l'invention, un cinquième groupe de composés est constitué par les composés pour lesquels R₂ représente un groupe méthyle ; A, L, B, R₇ et R₈ étant tels que définis ci-dessus.

Parmi les composés de formule générale (I) objets de l'invention, un sixième groupe de composés est constitué par les composés pour lesquels R₇ et R₈ représentent indépendamment l'un de l'autre, un atome d'hydrogène ou un groupe méthyle ;
A, L, B, et R₂ étant tels que définis ci-dessus.

Parmi les composés de formule générale (I) objets de l'invention, un septième groupe de composés est constitué par les composés pour lesquels :
- A représente un groupe C₁₋₇-alkylène éventuellement substitué par un ou deux groupes Rₐ ;
- B représente un groupe C₁₋₇-alkylène éventuellement substitué par un groupe R_{b} ;
- L représente un atome d'azote éventuellement substitué par un groupe R_{c} ou R_{d}, les atomes de carbone de A et de B étant éventuellement substitués par un ou plusieurs groupes R_{f} identiques ou différents l'un de l'autre ;
- deux groupes Rₐ peuvent former ensemble un groupe C₁₋₆-alkylène ;
- Rₐ et R_{b} peuvent former ensemble une liaison ou un groupe C₁₋₆-alkylène ;
- Rₐ et R_{c} peuvent former ensemble une liaison ou un groupe C₁₋₆-alkylène ;
- R_{b} et R_{c} peuvent former ensemble une liaison ou un groupe C₁₋₆-alkylène ;
- R_{d} représente un groupe choisi parmi l'atome d'hydrogène et les groupes C₁₋₆-alkyle, C₃₋₇-cycloalkyle, C₃₋₇-cycloalkyl-C₁₋₆-alkyle, hydroxy-C₁₋₆-alkyle, C₁₋₆-alkyloxy-C₁₋₆-alkyle, C₁₋₆-alkylthio-C-₁₋₆-alkyle, C₁₋₆-fluoroalkyle, benzyle ;
- R_{f} représente un groupe C₁₋₆-alkyle, C₃₋₇-cycloalkyle, C₃₋₇-cycloalkyle-C₁₋₆-alkyle, C₁₋₆-alkyloxy-C₁₋₆-alkyle, hydroxy-C₁₋₆-alkyle, C₁₋₆-fluoroalkyle ou phényle ;
- Rₐ, R_{b}, R_{c}, R₂, R₇ et R₈ étant tels que définis ci-dessus.

Parmi les composés de formule générale (I) objets de l'invention, un huitième groupe de composés est constitué par les composés pour lesquels :
- l'amine cyclique formée par -N-A-L-B- représente un groupe pipérazinyle, diazabicycloheptyle, hexahydropyrrolopyrrolyle, octahydro-pyrrolo-pyridinyle éventuellement substitué par un ou plusieurs groupes méthyle, isopropyle, butylène, phényle, benzyle, hydroxyméthyle, hydroxyéthyle, hydroxypropyle, hydroxy-méthyl-propyle, hydroxy-méthyl-butyle ;
- R₂, R₇ et R₈ étant tels que définis ci-dessus.

Parmi les composés de formule générale (I) objets de l'invention, un neuvième groupe de composés est constitué par les composés pour lesquels :
- l'amine cyclique formée par -N-A-L-B- représente un groupe (R)-3-méthylpipérazin-1-yle, 3,3-diméthylpipérazin-1-yle, (*cis*)-3,5-diméthylpipérazin-1-yle, 4-isopropyl-pipérazin-1-yle, 6,9-diaza-spiro[4.5]déc-9-yle, 3-phényl-pipérazin-1-yle, 4-benzyl-pipérazin-1-yle, 3-hydroxyméthyl-pipérazin-1-yle, 4-(2-hydroxy-éthyl)-pipérazin-1-yle, *(R*)-4-(2-hydroxy-propyl)-pipérazin-1-yle, (*S*)-4-(2-hydroxy-propyl)-pipérazin-1-yle, 4-(1-hydroxy-2-méthyl-propan-2-yl)-pipérazin-1-yle, 4-(2-hydroxy-2-méthyl-propyl)-pipérazin-1-yle, 4-(3-hydroxy-3-méthyl-butyl)-pipérazin-1-yle, (*R*)-3-phényl-pipérazin-1-yl, (*S*)-3-phényl-pipérazin-1-yle, 4-benzyl-pipérazin-1-yle, (*cis*)-5-méthyl-hexahydropyrrolo[3,4-c]pyrrol-2(1*H*)-yle, (*cis*)-5-(2-hydroxy-éthyl)-hexahydro-pyrrolo[3,4-*c*]pyrrol-2(1*H*)-yle, (*4aR*, *7aR*)-1-méthyl-octahydro-6*H-*pyrrolo[3,4-*b*]pyridin-6-yle, (*4aS*, *7aS*)-1-méthyl-octahydro-6*H*-pyrrolo[3,4-*b*]pyridin-6-yle, (1*S*,4*S*)-5-méthyl-2,5-diazabicyclo[2.2.1]hept-2-yle ;
- R₂, R₇ et R₈ étant tels que définis ci-dessus.

Parmi les composés de formule générale (I) objets de l'invention, un dixième groupe de composés est constitué par les composés pour lesquels :
- A représente un groupe C₁₋₇-alkylène éventuellement substitué par un ou deux groupes Rₐ;
- B représente un groupe C₁₋₇-alkylène éventuellement substitué par un groupe R_{b} ;
- L représente un atome de carbone substitué par deux groupes Rₑ₂ ;
   les atomes de carbone de A et de B étant éventuellement substitués par un ou plusieurs groupes R_{f} identiques ou différents l'un de l'autre ;
- Deux Rₑ₂ forment avec l'atome de carbone qui les porte une monoamine cyclique comportant éventuellement un atome d'oxygène, cette monoamine cyclique étant éventuellement substituée par un ou plusieurs groupes R_{f} identiques ou différents l'un de l'autre ;
- R_{f} représente un groupe C₁₋₆-alkyle ;
- Rₐ, R_{b}, R₂, R₇ et R₈ étant tels que définis ci-dessus.

Parmi les composés de formule générale (I) objets de l'invention, un onzième groupe de composés est constitué par les composés pour lesquels :
- l'amine cyclique formée par -N-A-L-B- représente un groupe diaza-spiro-undécyle ;
- R₂, R₇ et R₈ étant tels que définis ci-dessus

Parmi les composés de formule générale (I) objets de l'invention, un douzième groupe de composés est constitué par les composés pour lesquels :
- l'amine cyclique formée par -N-A-L-B- représente un groupe 2,9-diazaspiro[5.5]undéc-9-yle ;
- R₂, R₇ et R₈ étant tels que définis ci-dessus

Parmi les composés de formule générale (I) objets de l'invention, un treizième groupe de composés est constitué par les composés pour lesquels :
- A représente un groupe C₁₋₇-alkylène;
- B représente un groupe C₁₋₇-alkylène ;
- L représente un atome de carbone substitué par un groupe Rₑ₁ et un groupe R_{d} ;
- R_{d} représente un atome d'hydrogène ;
- Rₑ₁ représente un groupe -NR₄R₅ ou une monoamine cyclique comportant éventuellement un atome d'oxygène, la monoamine cyclique étant éventuellement substituée par un ou plusieurs R_{f} identiques ou différents l'un de l'autre ;
- R_{f} représente un groupe C₁₋₆-alkyle, C₃₋₇-cycloalkyle, C₃₋₇-cycloalkyle-C₁₋₆-alkyle ;
- R₂, R₇ et R₈ étant tels que définis ci-dessus.

Parmi les composés de formule générale (I) objets de l'invention, un quatorzième groupe de composés est constitué par les composés pour lesquels :
- A représente un groupe -C₂H₄- ;
- B représente un groupe -C₂H₄- ;
- L représente un atome de carbone substitué par un groupe Rₑ₁ et un groupe R_{d} ;
- R_{d} représente un atome d'hydrogène ;
- Rₑ₁ représente un groupement pyrrolidinyle ;
- R₂, R₇ et R₈ étant tels que définis ci-dessus.

Parmi les composés de formule générale (I) objets de l'invention, un quinzième groupe de composés est constitué par les composés pour lesquels :
- l'amine cyclique formée par -N-A-L-B- représente une 4-(pyrrolidin-1-yl)-pipéridin-1-yle ;
- R₂, R₇ et R₈ étant tels que définis ci-dessus.

Parmi les composés de formule générale (I) objets de l'invention, un seizième groupe de composés est constitué par les composés pour lesquels :
- R₂ représente un groupe méthyle ;
- L'amine cyclique formée par -N-A-L-B- représente un groupe (3*R*)-3-méthylpipérazin-1-yle ; 3,3-diméthyl-pipérazin-1-yle, (*cis*)-3,5-diméthyl-pipérazin-1-yle, 4-isopropyl-pipérazin-1-yle, (*cis*)-5-méthyl-hexahydro-pyrrolo[3,4-c]pyrrol-2(1*H*)-yle ;
- R₇ et R₈ représentent un atome d'hydrogène.

Parmi les composés de formule générale (I) objets de l'invention, un dix-septième groupe de composés est constitué par les composés pour lesquels :
- R₂ représente un 3-fluoro-phényle ou un 4-fluoro-phényle ;
- l'amine cyclique formée par -N-A-L-B- représente un groupe (*R*)-3-méthylpipérazin-1-yle, 3,3-diméthylpipérazin-1-yle, (*cis*)-3,5-diméthylpipérazin-1-yle, 4-isopropyl-pipérazin-1-yle, 6,9-diaza-spiro[4.5]déc-9-yle, 3-phényl-pipérazin-1-yle, 4-benzyl-pipérazin-1-yle, 3-hydroxyméthyl-pipérazin-1-yle, 4-(2-hydroxy-éthyl)-pipérazin-1-yle, *(R*)-4-(2-hydroxy-propyl)-pipérazin-1-yle, (*S*)-4-(2-hydroxy-propyl)-pipérazin-1-yle, 4-(1-hydroxy-2-méthyl-propan-2-yl)-pipérazin-1-yle, 4-(2-hydroxy-2-méthyl-propyl)-pipérazin-1-yle, 4-(3-hydroxy-3-méthyl-butyl)-pipérazin-1-yle, (*R*)-3-phényl-pipérazin-1-yl, (*S*)-3-phényl-pipérazin-1-yle, 4-benzyl-pipérazin-1-yle, (*cis*)-5-méthyl-hexahydropyrrolo[3,4-c]pyrrol-2(1*H*)-yle, (*cis*)-5-(2-hydroxy-éthyl)-hexahydro-pyrrolo[3,4-*c*]pyrrol-2(1*H*)-yle, (*4aR*, 7*aR*)-1-méthyl-octahydro-6*H-*pyrrolo[3,4-*b*]pyridin-6-yle, (*4aS*, *7aS*)-1-méthyl-octahydro-6*H*-pyrrolo[3,4-*b*]pyridin-6-yle, (1*S*,4*S*)-5-méthyl-2,5-diazabicyclo[2.2.1]hept-2-yle ;
- R₇ et R₈ représentent, indépendamment l'un de l'autre, un atome d'hydrogène ou un groupe méthyle.

Parmi les composés de formule générale (I) objets de l'invention, un dix-huitième groupe de composés est constitué par les composés pour lesquels :
- R₂ représente un groupe 4-fluoro-phényle ;
- l'amine cyclique formée par -N-A-L-B- représente un groupe 2,9-diazaspiro[5.5]undéc-9-yle ;
- R₇ et R₈ représentent un atome d'hydrogène.

Parmi les composés de formule générale (I) objets de l'invention, un dix-neuvième groupe de composés est constitué par les composés pour lesquels :
- R₂ représente un groupe 4-fluoro-phényle ;
- l'amine cyclique formée par -N-A-L-B- représente une 4-(pyrrolidin-1-yl)-pipéridin-1-yle ;
- R₇ et R₈ représentent un atome d'hydrogène.

Parmi les composés de formule générale (I) objets de l'invention, on peut notamment citer les composés suivants :
1. 2-Méthyl-6-[(*R*)-3-méthyl-pipérazin-1-yl]-3-(1*H*-pyrrolo[2,3-*b*]pyridin-4-yl)-imidazo[1,2-*b*]pyridazine ;
2. 6-(3,3-Diméthyl-pipérazin-1-yl)-2-méthyl-3-(1*H*-pyrrolo[2,3-*b*]pyridin-4-yl)-imidazo[1,2-*b*]pyridazine et son trichlorhydrate ;
3. 6-[(*cis*)-3,5-Diméthyl-pipérazin-1-yl]-2-méthyl-3-(1*H*-pyrrolo[2,3-*b*]pyridin-4-yl)-imidazo[1,2-*b*]pyridazine et son trichlorhydrate ;
4. 6-(4-Isopropyl-pipérazin-1-yl)-2-méthyl-3-(1*H*-pyrrolo[2,3-*b*]pyridin-4-yl)-imidazo[1,2-*b*]pyridazine et son trichlorhydrate ;
5. 2-Méthyl-6-[(*cis*)-5-méthyl-hexahydro-pyrrolo[3,4-*c*]pyrrol-2(1*H*)-yl]-3-(1*H-*pyrrolo[2,3-*b*]pyridin-4-yl)-imidazo[1,2-*b*]pyridazine et son trichlorhydrate ;
6. 2-(4-Fluoro-phényl)-6-[(*R*)-3-méthyl-pipérazin-1-yl]-3-(1*H*-pyrrolo[2,3-*b*]pyridin-4-yl)-imidazo[1,2-*b*]pyridazine ;
7. {4-[2-(4-Fluorophényl)-3-(1*H*-pyrrolo[2,3-*b*]pyridin-4-yl)-imidazo[1,2-*b*]pyridazin-6-yl]-pipérazin-2-yl}-méthanol ;
8. 6-(3,3-Diméthyl-pipérazin-1-yl)-2-(4-fluoro-phényl)-3-(1*H*-pyrrolo[2,3-*b*]pyridin-4-yl)-imidazo[1,2-*b*]pyridazine ;
9. 6-(3,3-Diméthyl-pipérazin-1-yl)-2-(3-fluorophényl)-8-méthyl-3-(1*H*-pyrrolo[2,3-*b*]pyridin-4-yl)-imidazo[1,2-*b*]pyridazine ;
10. 6-(3,3-Diméthyl-pipérazin-1-yl)-2-(4-fluorophényl)-8-méthyl-3-(1*H*-pyrrolo[2,3-*b*]pyridin-4-yl)-imidazo[1,2-*b*]pyridazine ;
11. 6-[(cis)-3,5-Diméthyl-pipérazin-1-yl]-2-(4-fluoro-phényl)-3-(1*H*-pyrrolo[2,3-*b*]pyridin-4-yl)-imidazo[1,2-*b*]pyridazine ;
12. 2-{4-[2-(3-Fluoro-phényl)-8-méthyl-3-(1*H*-pyrrolo[2,3-*b*]pyridin-4-yl)-imidazo[1,2-*b*]pyridazin-6-yl]-pipérazin-1-yl}-éthanol ;
13. 2-{4-[2-(4-Fluoro-phényl)-8-méthyl-3-(1*H*-pyrrolo[2,3-*b*]pyridin-4-yl)-imidazo[1,2-*b*]pyridazin-6-yl]-pipérazin-1-yl}-éthanol ;
14. 2-(4-Fluoro-phényl)-6-(4-isopropyl-pipérazin-1-yl)-3-(1*H*-pyrrolo[2,3-*b*]pyridin-4-yl)-imidazo[1,2-*b*]pyridazine ;
15. 2-(4-Fluoro-phényl)-6-(4-isopropyl-pipérazin-1-yl)-8-méthyl-3-(1*H*-pyrrolo[2,3-*b*]pyridin-4-yl)-imidazo[1,2-*b*]pyridazine ;
16. (*R*)-1-{4-[2-(4-Fluoro-phényl)-8-méthyl-3-(1*H*-pyrrolo[2,3-*b*]pyridin-4-yl)-imidazo[1,2-*b*]pyridazin-6-yl]-pipérazin-1-yl}-propan-2-ol
17. (*S*)-1-{4-[2-(4-Fluoro-phényl)-8-méthyl-3-(1*H*-pyrrolo[2,3-*b*]pyridin-4-yl)-imidazo[1,2-*b*]pyridazin-6-yl]-pipérazin-1-yl}-propan-2-ol
18. 6-(6,9-Diaza-spiro[4.5]déc-9-yl)-2-(4-fluoro-phényl)-8-méthyl-3-(1*H*-pyrrolo[2,3-*b*]pyridin-4-yl)-imidazo[1,2-*b*]pyridazine
19. 2-{4-[2-(4-Fluoro-phényl)-3-(1*H*-pyrrolo[2,3-*b*]pyridin-4-yl)-imidazo[1,2-*b*]pyridazin-6-yl]-pipérazin-1-yl}-2-méthyl-propan-1-ol
20. 1-{4-[2-(4-Fluoro-phényl)-3-(1*H*-pyrrolo[2,3-*b*]pyridin-4-yl)-imidazo[1,2-*b*]pyridazin-6-yl]-pipérazin-1-yl}-2-méthyl-propan-2-ol ;
21. 1-{4-[2-(3-Fluoro-phényl)-8-méthyl-3-(1*H*-pyrrolo[2,3-*b*]pyridin-4-yl)-imidazo[1,2-*b*]pyridazin-6-yl]-pipérazin-1-yl}-2-méthyl-propan-2-ol ;
22. 1-{4-[2-(4-Fluoro-phényl)-8-méthyl-3-(1*H*-pyrrolo[2,3-b]pyridin-4-yl)-imidazo[1,2-*b*]pyridazin-6-yl]-pipérazin-1-yl}-2-méthyl-propan-2-ol ;
23. 4-{4-[2-(4-Fluoro-phényl)-3-(1*H*-pyrrolo[2,3-*b*]pyridin-4-yl)-imidazo[1,2-*b*]pyridazin-6-yl]-pipérazin-1-yl}-2-méthyl-butan-2-ol ;
24. (*R*)-2-(4-Fluoro-phényl)-6-[3-phényl-pipérazin-1-yl]-3-(1*H*-pyrrolo[2,3-*b*]pyridin-4-yl)-imidazo[1,2-*b*]pyridazine et son trichlorhydrate ;
25. (*S*)-2-(4-Fluoro-phényl)-6-[3-phényl-pipérazin-1-yl]-3-(1*H*-pyrrolo[2,3-*b*]pyridin-4-yl)-imidazo[1,2-*b*]pyridazine et son trichlorhydrate ;
26. 2-(4-Fluoro-phényl)-8-méthyl-6-[3-phényl-pipérazin-1-yl]-3-(1*H*-pyrrolo[2,3-*b*]pyridin-4-yl)-imidazo[1,2-*b*]pyridazine ;
27. 6-(4-Benzyl-pipérazin-1-yl)-2-(4-fluoro-phényl)-3-(1*H*-pyrrolo[2,3-*b*]pyridin-4-yl)-imidazo[1,2-*b*]pyridazine ;
28. (*cis*)-2-(4-Fluoro-phényl)-6-(5-méthyl-hexahydro-pyrrolo[3,4-c]pyrrol-2(1*H*)-yl)-3-(1*H-*pyrrolo[2,3-*b*]pyridin-4-yl)-imidazo[1,2-*b*]pyridazine ;
29. (*cis*)-2-(4-Fluoro-phényl)-8-méthyl-6-(5-méthyl-hexahydro-pyrrolo[3,4-c]pyrrol-2(1*H*)-yl)-3-(1*H*-pyrrolo[2,3-*b*]pyridin-4-yl)-imidazo[1,2-*b*]pyridazine ;
30. (*cis*)-2-{5-[2-(4-Fluoro-phényl)-3-(1*H*-pyrrolo[2,3-*b*]pyridin-4-yl)-imidazo[1,2-*b*]pyridazin-6-yl]-hexahydro-pyrrolo[3,4-*c*]pyrrol-2(1*H*)-yl}-éthanol ;
31. (*cis*)-2-{5-[2-(4-Fluoro-phényl)-8-méthyl-3-(1*H*-pyrrolo[2,3-*b*]pyridin-4-yl)-imidazo[1,2-*b*]pyridazin-6-yl]-hexahydro-pyrrolo[3,4-*c*]pyrrol-2(1*H*)-yl}-éthanol ;
32. 2-(4-Fluoro-phényl)-8-méthyl-6-((*4aR*,*7aR*)-1-méthyl-octahydro-6*H*-pyrrolo[3,4-*b*]pyridin-6-yl)-3-(1*H*-pyrrolo[2,3-*b*]pyridin-4-yl)-imidazo[1,2-*b*]pyridazine
33. 2-(4-Fluoro-phényl)-8-méthyl-6-((*4aS*,*7aS*)-1-méthyl-octahydro-6*H*-pyrrolo[3,4-*b*]pyridin-6-yl)-3-(1*H*-pyrrolo[2,3-*b*]pyridin-4-yl)-imidazo[1,2-*b*]pyridazine
34. 2-(4-Fluoro-phényl)-6-((*1S*,*4S*)-5-méthyl-2,5-diaza-bicyclo[2.2.1]hept-2-yl)-3-(1*H-*pyrrolo[2,3-*b*]pyridin-4-yl)-imidazo[1,2-*b*]pyridazine ;
35. 9-[2-(4-Fluoro-phényl)-3-(1*H*-pyrrolo[2,3-*b*]pyridin-4-yl)-imidazo[1,2-*b*]pyridazin-6-yl]-2,9-diaza-spiro[5.5]undécane ;
36. 2-(4-Fluoro-phényl)-6-(4-pyrrolidin-1-yl-pipéridin-1-yl)-3-(1*H*-pyrrolo[2,3-*b*]pyridin-4-yl)-imidazo[1,2-*b*]pyridazine.

L'invention a également pour objet un procédé de préparation des composés de l'invention de formule (I).

Conformément à l'invention, on peut préparer les composés de formule générale (I) selon le procédé général décrit dans le schéma 1 ci-après.

De manière générale et comme illustré dans le schéma 1, les dérivés de 6-cycloamino-3-(1*H*-pyrrolo[2,3-*b*]pyridin-4-yl)imidazo[1,2-*b*]pyridazine, de formule générale (I) dans laquelle R₂, A, L, B, R₇ et R₈ sont tels que définis ci-dessus peuvent être préparés à partir d'un dérivé de (1*H*-pyrrolo[2,3-*b*]pyridin-4-yl)imidazo[1,2-*b*]pyridazine de formule générale (II), dans laquelle R₂, R₇ et R₈ sont tels que définis ci-dessus et X₆ représente un groupe partant tel qu'un halogène, par traitement au moyen d'une amine de formule générale (III) dans laquelle A, L et B sont tels que définis précédemment. Cette réaction peut être effectuée par chauffage des réactifs dans un solvant polaire tel que le pentanol ou le diméthylsulfoxyde.

Les dérivés de (1*H*-pyrrolo[2,3-*b*]pyridin-4-yl)imidazo[1,2-*b*]pyridazine de formule générale (II) tels que définis ci-dessus peuvent être obtenus à partir de dérivés de formule générale (IV) dans laquelle R₂, X₆, R₇ et R₈ sont tels que définis ci-dessus et GP représente un groupe protecteur de fonction amine tel qu'un sulfonate, par exemple le tosylate ou tout autre groupement usuellement utilisé pour la protection d'imidazole, de pyrrole ou d'indole (« Protective groups in organic chemistry », T, W.Greene and P., G., M. Wuts, 2rd Edition, Wiley Interscience, p. 385-397). La transformation des dérivés de formule générale (IV) est alors effectuée par une réaction de déprotection, par exemple, par traitement au moyen d'une base telle que de la soude lorsque GP représente un groupe benzène ou toluène sulfonyle.

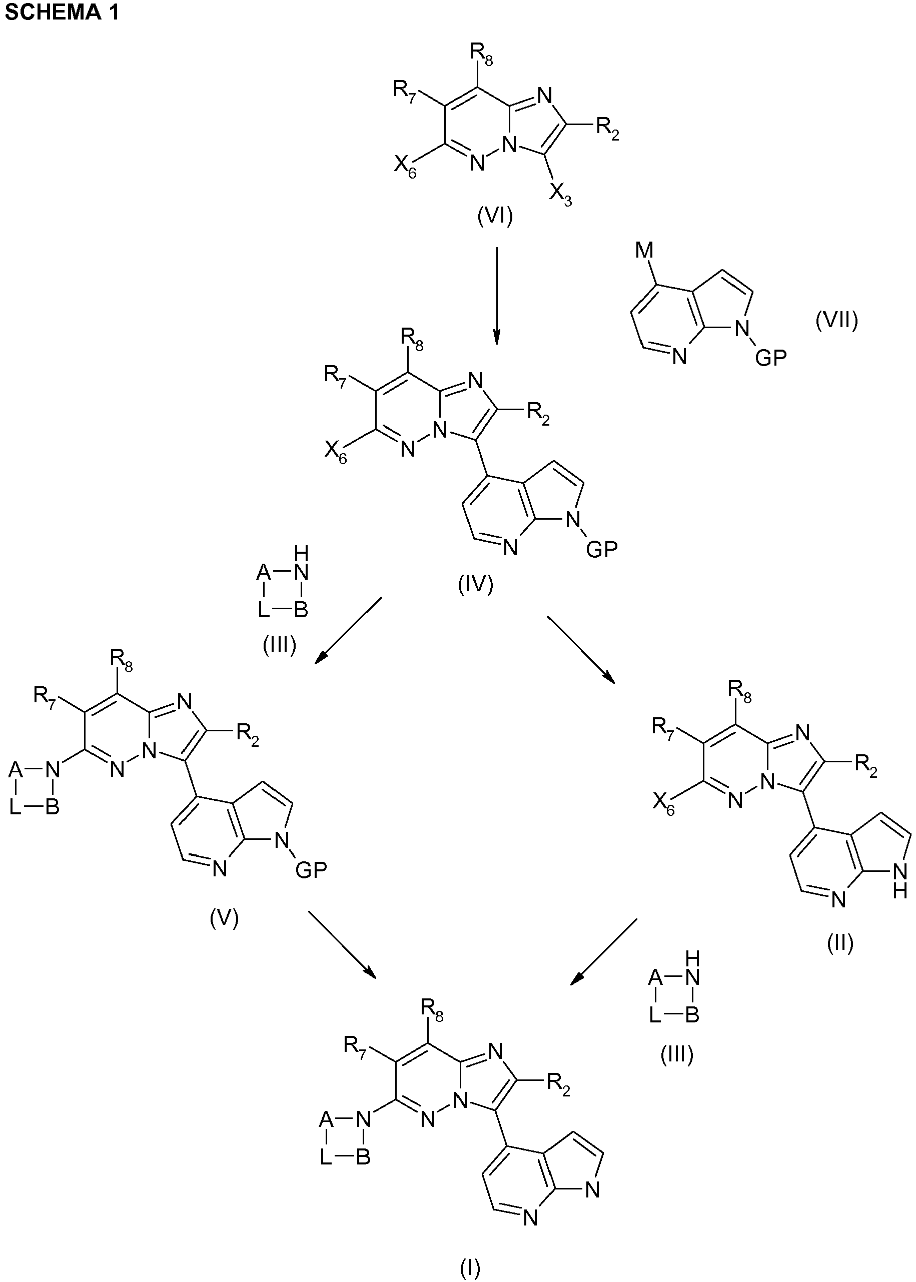

De façon alternative, les dérivés de 6-cycloamino-3-(1*H*-pyrrolo[2,3-*b*]pyridin-4-yl)imidazo[1,2-*b*]pyridazine de formule générale (I) peuvent également être préparés par déprotection d'un dérivé (1*H*-pyrrolo[2,3-*b*]pyridin-4-yl)imidazo[1,2-*b*]pyridazine de formule générale (V) dans laquelle R₂, A, L, B, R₇, R₈ et GP sont tels que définis ci-dessus. La transformation des dérivés de formule générale (V) est alors effectuée par une réaction de déprotection, par exemple, par traitement au moyen d'une base telle que de la soude lorsque GP représente un groupe benzène ou toluène sulfonyle.

Les dérivés de formule générale (V) peuvent être préparés à partir de dérivés de formule générale (IV) tels que définis ci-dessus par traitement au moyen d'une amine de formule générale (III) dans laquelle A, L et B sont tels que définis précédemment. Cette réaction peut être effectuée par chauffage des réactifs dans un solvant polaire tel que le pentanol ou le diméthylsulfoxyde.

Les dérivés de (1*H*-pyrrolo[2,3-*b*]pyridin-4-yl)imidazo[1,2-*b*]pyridazine de formule générale (IV), dans laquelle R₂, X₆, R₇, R₈ et GP sont tels que définis ci-dessus peuvent être préparés par couplage métallocatalysé selon les conditions de Suzuki entre un dérivé de 3-halogéno-imidazo[1,2-*b*]pyridazine de formule générale (VI) dans laquelle R₂, X₆, R₇ et R₈ sont tels que définis ci-dessus tandis que X₃ représente un atome de brome ou d'iode avec un dérivé de 1*H*-pyrrolo[2,3-*b*]pyridine de formule générale (VII) dans laquelle GP est tel que défini ci-dessus et M représente un groupe dihydroxyboryle ou dialkyloxyboryle, le plus fréquemment un groupe 4,4,5,5-tétraméthyl-1,3,3,2-dioxaborolan-2-yle.

Les couplages selon la méthode de Suzuki sont par exemple réalisés par chauffage en présence d'un catalyseur tel que le 1,1'-bis (diphénylphosphino)ferrocènedichloropalladium, d'une base minérale telle que le carbonate de césium, dans un mélange de solvants tels que le dioxane et l'eau.

Les dérivés de 3-halogéno-1*H*-pyrrolo[2,3-*b*]pyridin-4-yl)imidazo[1,2-*b*]pyridazine de formule générale (VI) et les dérivés de 1*H*-pyrrolo[2,3-*b*]pyridine de formule générale (VII) tels que définis ci-dessus sont connus ou peuvent être préparés selon des méthodes connues de l'homme de métier.

Dans certains cas, les dérivés de 6-cycloamino-3-(1*H*-pyrrolo[2,3-*b*]pyridin-4-yl)imidazo[1,2-b]pyridazine de formule générale (I) pour lesquels l'amine formée par N, L, A et B comporte une deuxième amine secondaire ou tertiaire peuvent être préparés respectivement à partir de l'amine primaire ou secondaire correspondante par alkylation ou amino-réduction selon des méthodes usuelles pour l'homme du métier.

### Groupes protecteurs

Dans certains cas, les dérivés de formules générales (I) ou (V) telles que définies ci-dessus avec un groupe N-A-L-B comportant une fonction amine primaire ou secondaire, peuvent être protégés lors de la synthèse sur cette fonction amine primaire ou secondaire par un groupe protecteur, par exemple un benzyle ou un t-butyloxycarbonyle.

Les produits de structure générale (I) tels que définis ci-dessus sont alors obtenus selon les procédés décrits après une étape supplémentaire de déprotection du groupe protecteur selon les conditions usuelles connues de l'homme du métier.

### Groupes partants

Dans ce qui précède, on entend par groupe partant un groupe pouvant être facilement clivé d'une molécule par rupture d'une liaison hétérolytique, avec départ d'une paire électronique. Ce groupe peut, par exemple, être ainsi remplacé facilement par un autre groupe lors d'une réaction de substitution. De tels groupes partants sont, par exemple, les halogènes ou un groupe hydroxy activé tel qu'un mésyle, tosyle, triflate, acétyle, etc. Des exemples de groupes partants ainsi que des références pour leurs préparations sont données dans « Advances in Organic Chemistry», J. March, 3rd Edition, Wiley Interscience, p. 310-316.

Les exemples suivants décrivent la préparation de certains composés conformes à l'invention. Ces exemples ne sont pas limitatifs et ne font qu'illustrer l'invention. Les numéros des composés exemplifiés renvoient à ceux donnés dans le tableau 1, ci-après, qui illustrent les structures chimiques et les propriétés physiques, respectivement de quelques composés selon l'invention.

### Exemple 1 (composé n° 29) : (Cis)-2-(4-fluoro-phényl)-8-méthyl-6-(5-méthyl-hexahydro-pyrrolo[3,4-c]pyrrol-2(1H)-yl)-3-(1H-pyrrolo[2,3-b]pyridin-4-yl)-imidazo[1,2-b]pyridazine

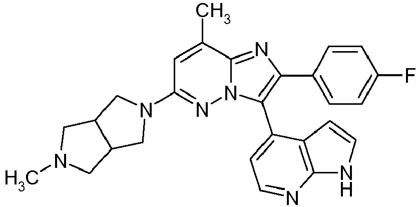

### Etape 1.1 6-Chloro-4-méthyl-pyridazin-3-ylamine et 6-chloro-5-méthyl-pyridazin-3-ylamine

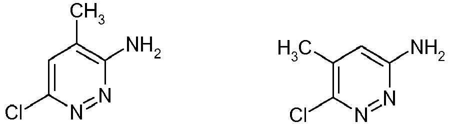

Un mélange de 50,0 g (307 mmoles) de 3,6-dichloro-4-méthylpyridazine dans 170 ml d'ammoniaque (30%) est chauffé à 120°C pendant 16 h dans un réacteur en acier à la pression interne de 10 bars.

Le réacteur est refroidit et le mélange réactionnel est versé dans 200 ml d'eau. Le solide formé est isolé par filtration et séché sous vide pour donner 38,7 g d'un mélange contenant environ 45% de 6-chloro-4-méthyl-pyridazin-3-ylamine (CAS 64068-00-4) et 55% de 6-chloro-5-méthyl-pyridazin-3-ylamine (CAS 66346-87-0).

RMN ¹H (CDCl₃) δ : 7,20 et 6,75 (2s, 1H) ; (d, 0,55H) ; 4,9 (sl, 2H) ; 2,40 et 2,25 (2s, 3H) ppm.

### Etape 1.2 6-Chloro-2-(4-fluoro-phényl)-8-méthyl-imidazo[1,2-b]pyridazine et 6-chloro-2-(4-fluoro-phényl)-7-méthyl-imidazo[1,2-b]pyridazine

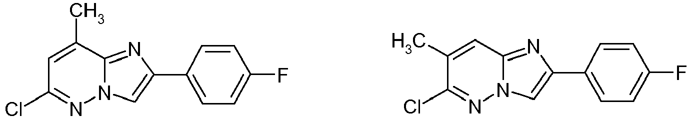

Le mélange de 76 g (350 mmoles) de 2-bromo-1-(4-fluoro-phényl)-éthanone (CAS 403-29-2) avec 38,7 g (269 mmoles) du mélange de 6-chloro-4-méthyl-pyridazin-3-ylamine et de 6-chloro-5-méthyl-pyridazin-3-ylamine obtenu à l'étape 1.1 dans 500 ml de n-butanol est chauffé à 120°C pendant 18 heures.

Le solvant est éliminé par évaporation sous pression réduite et le solide trituré dans de l'acétone. Après glaçage, le solide est séparé par filtration. Le filtrat est concentré sous pression réduite et le résidu trituré dans de l'éther diéthylique. Après glaçage, le solide est à nouveau séparé par filtration. Les deux lots de solide (75 g) sont joints et dissous dans 1 l d'eau. La solution est basifiée par addition d'ammoniaque et le produit est extrait avec du chloroforme. La phase organique est séchée sur sulfate de sodium et le solvant est évaporé sous pression réduite pour donner un solide rouge-brun. La séparation des deux isomères est réalisée par chromatographie sur colonne de gel de silice (2x800 g) en éluant avec du dichlorométhane. On obtient 21,9 g de 6-chloro-2-(4-fluoro-phényl)-8-méthyl-imidazo[1,2-*b*]pyridazine sous forme de solide beige après trituration dans l'éther isopropylique, glaçage, filtration et séchage.

PF : 210-212°C

RMN ¹H (CDCl₃) δ : 8,20 (s, 1 H) ; 8,00 (dd, 2H) ; 7,25 (pt, 2H) ; 6,95 (s, 1 H) ; 2,75 (s, 3H) ppm.

La poursuite de l'élution avec un mélange de 2 % de méthanol dans le dichlorométhane donne 22,0 g de 6-chloro-2-(4-fluoro-phényl)-7-méthyl-imidazo[1,2-*b*]pyridazine sous forme de solide beige après trituration dans l'éther isopropylique, glaçage, filtration et séchage.

PF : 196-198°C

RMN ¹H (CDCl₃) δ : 8,15 (s, 1H) ; 8,00 (dd, 2H) ; 7,80 (s, 1H) ; 7,20 (pt, 2H) ; 2,55 (s, 3H) ppm.

### Etape 1.3 6-Chloro-2-(4-fluoro-phényl)-3-iodo-8-méthyl-imidazo[1,2-b]pyridazine

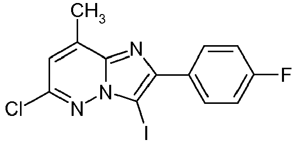

A une suspension de 21,9 g (83,7 mmoles) de 6-chloro-2-(4-fluoro-phényl)-8-méthyl-imidazo[1,2-*b*]pyridazine dans 500 ml de chloroforme sont ajoutés 20,4 g (126 mmoles) de monochlorure d'iode en solution dans 40 à 50 ml de méthanol. Après 2 heures d'agitation à température ambiante, 5,0 g (31 mmoles) de monochlorure d'iode en solution dans environ 10 ml de méthanol sont de nouveau ajoutés.

Après 2 heures additionnelles d'agitation, la solution est versée sur 500 ml d'une solution aqueuse d'hydrogénocarbonate de sodium et le mélange est traité sous forte agitation avec du thiosulfate de sodium qui est ajouté par portion jusqu'à décoloration du mélange (rouge à jaune).

La phase organique est séparée, séchée sur sulfate de sodium et le solvant éliminé par évaporation sous pression réduite. Le solide obtenu, est alors trituré dans de l'acétonitrile, la suspension est glacée et le solide est isolé par filtration pour donner 30,7 g de 6-chloro-2-(4-fluoro-phényl)-3-iodo-8-méthyl-imidazo[1,2-*b*]pyridazine sous forme de poudre beige.

PF : 190-192°C

RMN ¹H (CDCl₃) δ : 8,05 (dd, 2H) ; 7,10 (pt, 2H) ; 6,90 (s, 1H) ; 2,65 (s, 3H) ppm.

### Etape 1.4 6-Chloro-2-(4-fluorophényl)-8-méthyl-3-[1-(phénylsulfonyl)-1H-pyrrolo[2,3-b]pyridin-4-yl]imidazo[1,2-b]pyridazine

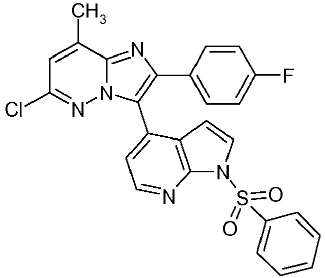

A un mélange préalablement dégazé et sous argon de 5,00 g (12,9 mmoles) de 6-chloro-2-(4-fluoro-phényl)-3-iodo-8-méthyl-imidazo[1,2-*b*]pyridazine, de 5,95 g (15,5 mmoles) de 1-(phénylsulfonyl)-4-(4,4,5,5-tétraméthyl-1,3,2-dioxaborolan-2-yl)-1*H*-pyrrolo[2,3-*b*]pyridine (CAS 942919-24-6) et de 12,6 g (38,7 mmoles) de carbonate césium dans 50 ml d'un mélange de tétrahydrofurane et d'eau (9/1) on additionne 0,95 g (1,2 mmole) de complexe de dichlorure 1,1'-bis(diphénylphosphino)ferrocène-palladium(II) et de dichlorométhane.

Le mélange est chauffé à reflux pendant 18 heures, puis versé dans 300 ml d'eau. Le produit est extrait avec du dichlorométhane. La phase organique est séparée, séchée sur sulfate de sodium et le solvant éliminé par évaporation sous pression réduite. Le solide marron obtenu est alors chromatographié sur colonne de gel de silice (200 g) en éluant avec un mélange de dichlorométhane, de méthanol et d'ammoniaque (97/3/0,3) pour donner 5,99 g de 6-chloro-2-(4-fluoro-phényl)-8-méthyl-3-[1-(phénylsulfonyl)-1*H-*pyrrolo[2,3-*b*]pyridin-4-yl]-imidazo[1,2-*b*]pyridazine sous forme de poudre jaune après trituration dans l'éther isopropylique, glaçage, filtration et séchage.

PF : 226-228°C

RMN ¹H (CDCl₃) δ : 8,65 (d, 1 H) ; 8,30 (d, 2H) ; 7,6 (m, 7H) ; 7,05 (m, 3H) ; 6,10 (d, 1H) ; 2,80 (s, 3H) ppm.

### Etape 1.5 6-Chloro-2-(4-fluorophényl)-8-méthyl-3-(1H-pyrrolo[2,3-b]pyridin-4-yl)imidazo[1,2-b]pyridazine

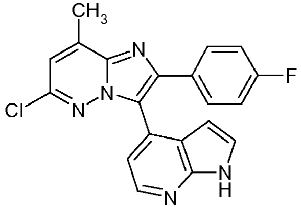

A une suspension de 0,50 g (0,97 mmole) de 6-chloro-2-(4-fluoro-phényl)-8-méthyl-3-[1-(phénylsulfonyl)-1*H*-pyrrolo[2,3-*b*]pyridin-4-yl]-imidazo[1,2-*b*]pyridazine dans 10 ml d'un mélange de méthanol et quelques ml de tétrahydrofurane, on additionne 0,32 ml (1,9 mmole) d'une solution aqueuse 6N de soude. Le mélange devient progressivement homogène et la réaction est agitée pendant 30 minutes. Le milieu réactionnel est dilué avec 100 ml d'eau et le produit est extrait avec du dichlorométhane. La phase organique est séparée, séchée sur sulfate de sodium et le solvant éliminé par évaporation sous pression réduite. Le solide orange obtenu est alors chromatographié sur colonne de gel de silice (35 g) en éluant avec un mélange de dichlorométhane, de méthanol et d'ammoniaque (95/5/0,5) pour donner 0,293 g de 6-chloro-2-(4-fluorophényl)-8-méthyl-3-(1*H*-pyrrolo[2,3-*b*]pyridin-4-yl)imidazo[1,2-*b*]pyridazine sous forme de poudre jaune après trituration dans l'éther isopropylique, glaçage, filtration et séchage.

PF : 226-228°C

RMN ¹H (CDCl₃) δ : 9,5 (sl, 1 H) ; 8,40 (d, 1 H) ; 7,55 (d, 2H) ; 7,30 (d, 1 H) ; 7,2 (m, 1 H) ; 6,9 (m, 3H) ; 5,90 (m, 1 H) ; 2,70 (s, 3H) ppm.

### Etape 1.6 (Cis)-2-(4-Fluorophényl)-8-méthyl-6-[(cis)-5-méthylhexahydropyrrolo[3,4-c]pyrrol-2(1H)-yl]-3-(1H-pyrrolo[2,3-b]pyridin-4-yl)imidazo[1,2-b]pyridazine

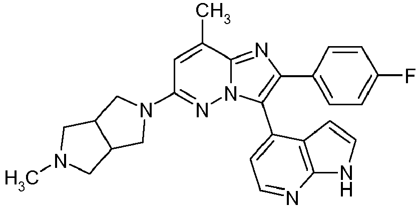

Dans un tube scellé, le mélange de 0,29 g (0,77 mmole) de 6-chloro-2-(4-fluorophényl)-8-méthyl-3-(1*H*-pyrrolo[2,3-*b*]pyridin-4-yl)imidazo[1,2-*b*]pyridazine, de 0,12 g (0,92 mmole) de (*cis*)-octahydro-6*H*-2-méthyl-pyrrolo[3,4-*c*]pyrrole (CAS 172739-03-6) et de 0,11 ml (0,77 mmole) de triéthylamine dans 4 ml de pentanol est chauffé à 150°C pendant 26 heures. Après refroidissement, le milieu réactionnel est versé dans 60 ml d'une solution aqueuse d'acide chlorhydrique 1N et la solution est lavée avec de l'acétate d'éthyle. La phase aqueuse est alors basifiée par addition d'ammoniaque et le produit est extrait avec du dichlorométhane. La phase organique est séparée, séchée sur sulfate de sodium et le solvant éliminé par évaporation sous pression réduite. L'huile marron obtenue est alors chromatographiée sur colonne de gel de silice (35 g) en éluant avec un mélange de dichlorométhane, de méthanol et d'ammoniaque (90/10/1) pour donner 0,101 g de 2-(4-fluorophényl)-8-méthyl-6-[(*cis*)-5-méthylhexahydropyrrolo[3,4-*c*]pyrrol-2(1*H*)-yl]-3-(1*H-*pyrrolo[2,3-*b*]pyridin-4-yl)imidazo[1,2-*b*]pyridazine sous forme de poudre beige après trituration dans l'éther diéthylique, glaçage, filtration et séchage.

PF : 255°C (décomposition)

RMN ¹H (DMSO d₆) δ : 11,7 (s, 1 H) ; 8,35 (d, 1 H) ; 7,50 (m, 2H) ; 7,40 (d, 1 H) ; 7,30 (d, 1 H) ; 7,1 (pt, 2H) ; 6,90 (m, 1 H) ; 5,90 (d, 1 H) ; 3,50 (m, 2H) ; 3,20 (dd, 2H) ; 2,85 (m ,2H) ; 2,60 (s, 3H) ; 2,45 (m, 2H) ; 2,40 (m, 2H) ; 2,20 (s, 3H) ppm.

### Exemple 2 (composé n° 1) : 2-Méthyl-6-[(R)-3-méthyl-pipérazin-1-yl]-3-(1H-pyrrolo[2,3-b]pyridin-4-yl)imidazo[1,2-b]pyridazine

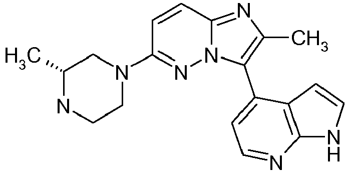

### Etape 2.1. 6-Chloro-3-iodo-2-méthyl-imidazo[1,2-b]pyridazine

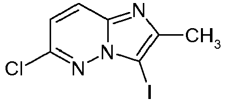

A une solution de 7,00 g (41,8 mmoles) de 6-chloro-2-méthyl-imidazo[1,2-*b*]pyridazine (CAS 14793-00-1) dans 300 ml de chloroforme refroidie à 0°C, on ajoute 10,2 g (62,7 mmoles) de monochlorure d'iode en solution dans 20 ml de méthanol. La réaction est ensuite laissée à température ambiante pendant 16 heures puis versée sur un mélange d'une solution de thiosulfate de sodium 5% et d'hydrogénocarbonate de sodium. Le produit est extrait avec du dichlorométhane, la phase organique est séchée sur sulfate de sodium et le solvant évaporé sous pression réduite.

Le résidu solide est trituré avec de l'acétonitrile puis isolé par filtration pour donner après séchage 8,5 g de 6-chloro-3-iodo-2-méthyl-imidazo[1,2-*b*]pyridazine sous forme d'un solide jaune.

RMN ¹H (CDCl₃) δ : 7,80 (d, 1H) ; 7,10 (d, 1H) ; 2,55 (s, 3H) ppm.

### Etape 2.2. 6-Chloro-2-méthyl-3-{1-[(4-méthylphényl)sulfonyl]-1H-pyrrolo[2,3-b]pyridin-4-yl}imidazo[1,2-b]pyridazine

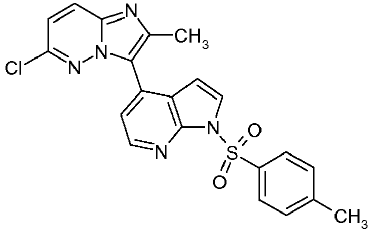

A un mélange préalablement dégazé et sous argon de 0,470 g (1,60 mmole) de 6-chloro-3 iodo-2-méthyl-imidazo[1,2-*b*]pyridazine, de 0,765 g (1,92 mmole) de 1-[(4-méthylphényl)sulfonyl]-4-(4,4,5,5-tétraméthyl-1,3,2-dioxaborolan-2-yl)-1*H*-pyrrolo[2,3-b]pyridine (CAS 916176-50-6) et de 1,56 g (4,80 mmoles) de carbonate césium dans 10 ml d'un mélange de tétrahydrofurane et d'eau (9/1), on additionne 0,12 g (0,14 mmole) de complexe de dichlorure 1,1'-bis(diphénylphosphino)ferrocène-palladium(II) et de dichlorométhane. Le mélange est chauffé à reflux pendant 18 heures, puis versé dans 100 ml d'eau. Le produit est extrait avec du dichlorométhane. La phase organique est séparée, séchée sur sulfate de sodium et le solvant éliminé par évaporation sous pression réduite. Le solide marron obtenu est alors chromatographié sur colonne de gel de silice greffée de type aminopropyle *(SiNH2 ;* 30 g), en éluant avec un mélange de dichlorométhane et d'éther de pétrole (70/30) pour donner 0,42 g de 6-chloro-2-méthyl-3-{1-[(4-méthylphényl)sulfonyl]-1*H*-pyrrolo[2,3-*b*]pyridin-4-yl}imidazo[1,2-*b*]pyridazine sous forme de poudre blanche.

PF: 138-140°C

RMN ¹H (CDCl₃) δ : 8,50 (d, 1H) ; 8,10 (d, 2H) ; 7,85 (d, 1H) ; 7,75 (d, 1H) ; 7,25 (d, 2H) ; 7,05 (d, 1 H) ; 6,30 (d, 1 H) ; 2,45 (s, 3H) ; 2,35 (s, 3H) ppm.

### Etape 2.3. 2-Méthyl-6-[(R)-3-méthylpipérazin-1-yl]-3-{1-[(4-méthylphényl)sulfonyl]-1H-pyrrolo[2,3-b]pyridin-4-yl}imidazo[1,2-b]pyridazine

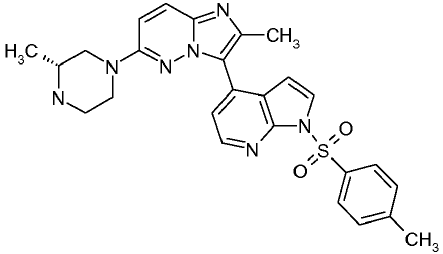

Un mélange de 0,325 g (0,97 mmole) de 6-chloro-2-méthyl-3-{1-[(4-méthylphényl)sulfonyl]-1*H*-pyrrolo[2,3-*b*]pyridin-4-yl}imidazo[1,2-*b*]pyridazine, de 0,15 g (1,5 mmole) de (2*R*)-2-méthylpipérazine et de 0,10 ml (0,74 mmole) de triéthylamine dans 5 ml de pentanol est chauffé à reflux pendant 3 jours à 150°C. Le milieu réactionnel est dilué avec 100 ml d'une solution aqueuse d'acide chlorhydrique 1N et la solution est lavée avec de l'acétate d'éthyle. La phase aqueuse est alors basifiée par addition d'ammoniaque et le produit est extrait avec du dichlorométhane. La phase organique est séparée, séchée sur sulfate de sodium et le solvant éliminé par évaporation sous pression réduite. L'huile marron obtenue est alors chromatographiée sur colonne de gel de silice (35 g) en éluant avec un mélange de dichlorométhane, de méthanol et d'ammoniaque (90/10/1) pour donner 0,293 g de 2-méthyl-6-[(3*R*)-3-méthylpipérazin-1-yl]-3-{1-[(4-méthylphényl)sulfonyl]-1*H*-pyrrolo[2,3-*b*]pyridin-4-yl}imidazo[1,2-*b*]pyridazine sous forme d'huile jaune après séchage.

RMN ¹H (CDCl₃) δ : 8,60 (d, 1 H) ; 8,20 (d, 2H) ; 7,80 (d, 1 H) ; 7,75 (d, 1 H) ; 7,40 (s, 1H) ; 7,35 (d, 2H) ; 6,90 (d, 1 H) ; 6,55 (d, 1 H) ; 3,8 (m, 2H) ; 2,9 (m, 1 H) ; 2,7 (m, 3H) ; 2,40 (s, 3H) ; 2,35 (m, 1 H) ; 2,25 (sl, 1 h) ; 0,95 (d, 3H) ppm.

### Etape 2.4. 2-Méthyl-6-[(R)-3-méthylpipérazin-1-yl]-3-(1H-pyrrolo[2,3-b]pyridin-4-yl)imidazo[1,2-b]pyridazine

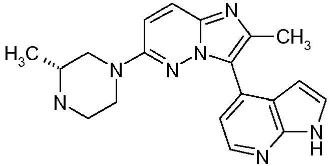

A une solution de 0,300 g (0,60 mmole) de 2-méthyl-6-[(3*R*)-3-méthylpipérazin-1-yl]-3-{1-[(4-méthylphényl)sulfonyl]-1*H*-pyrrolo[2,3-*b*]pyridin-4-yl}imidazo[1,2-*b*]pyridazine dans 5 ml de méthanol, on additionne 0,20 ml (1,6 mmole) d'une solution aqueuse 6N de soude.

Le mélange est chauffé à 60°C pendant 1 heure puis versé dans 100 ml d'eau. Le produit est extrait avec du dichlorométhane. La phase organique est séparée, séchée sur sulfate de sodium et le solvant est éliminé par évaporation sous pression réduite. Le résidu obtenu est alors chromatographié sur colonne de gel de silice (15 g) en éluant avec un mélange de dichlorométhane, de méthanol et d'ammoniaque (90/10/1) pour donner 0,195 g de 2-méthyl-6-[(3*R*)-3-méthylpipérazin-1-yl]-3-(1*H*-pyrrolo[2,3-*b*]pyridin-4-yl)imidazo[1,2-*b*]pyridazine après trituration dans l'éther diisopropylique, glaçage, filtration et séchage.

PF : 202-204°C

[alpha]_{D} = + 29,0° (CH₃OH, c= 0,683 g/100ml)

RMN ¹H (DMSO d₆) δ : 8,35 (d, 1 H) ; 7,80 (d, 1 H) ; 7,50 (d, 1 H) ; 7,30 (d, 1 H) ; 7,20 (d, 1H) ; 6,30 (d, 1 H) ; 3,85 (m, 2H) ; 2,9 (m, 1 H) ; 2,7 (m, 3H) ; 2,40 (s, 3H) ; 2,35 (m, 1 H) ; 2,2 (sl, 1 H) ; 0,95 (d, 3H) ppm.

### Exemple 3 (composé n° 6) : 2-(4-Fluorophényl)-6-[(3R)-3-méthylpipérazin-1-yl]-3-(1H-pyrrolo[2,3-b]pyridin-4-yl)imidazo[1,2-b]pyridazine

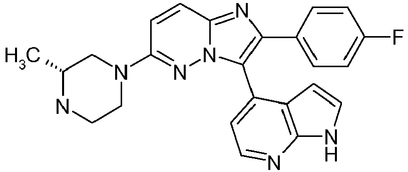

### Etape 3.1. 6-Chloro-2-(4-fluoro-phényl)-3-iodo-imidazo[1,2-b]pyridazine

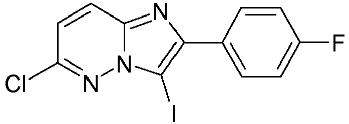

A une solution refroidie à 0°C de 5,20 g (21,0 mmoles) de 6-chloro-2-(4-fluoro-phényl)-imidazo[1,2-*b*]pyridazine (numéro CAS : 244081-70-7) dans 130 ml de chloroforme, on additionne au goutte à goutte rapide une solution de 6,61 g (40,9 mmoles) de monochlorure d'iode dans 40 ml de chloroforme. Après retour à la température ambiante et 4 heures d'agitation, le mélange est traité avec une solution aqueuse à 5% de thiosulfate de sodium. Le produit est extrait avec du dichlorométhane, la phase organique est séchée par filtration sur cartouche filtrante hydrophobe et concentrée sous pression réduite. Le résidu est trituré dans de l'acétonitrile, le solide est isolé après filtration et rinçage avec de l'éther diisopropylique. On isole 5,7 g de poudre beige après séchage sous vide.

PF : 215°C

RMN ¹H (DMSOd₆) δ : 8,20 (m; 3H), 7,40 (m, 3H) ppm.

### Etape 3.2. 6-Chloro-2-(4-fluorophényl)-3-{1-[(4-méthylphényl)sulfonyl]-1H-pyrrolo[2,3-b]pyridin-4-yl}imidazo[1,2-b]pyridazine

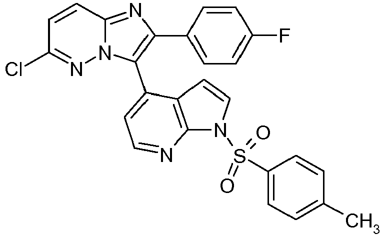

A un mélange préalablement dégazé et sous argon de 0,782 g (2,09 mmoles) de 6-chloro-2-(4-fluoro-phényl)-3-iodo-imidazo[1,2-*b*]pyridazine, de 1,00 g (2,51 mmoles) de 1-[(4-méthylphényl)sulfonyl]-4-(4,4,5,5-tétraméthyl-1,3,2-dioxaborolan-2-yl)-1H-pyrrolo[2,3-b]pyridine (CAS 916176-50-6) et de 2,05 g (6,28 mmoles) de carbonate césium dans 15 ml d'un mélange de tétrahydrofurane et d'eau (9/1), on additionne 0,15 g (0,19 mmole) de complexe de dichlorure 1,1'-bis(diphénylphosphino)ferrocène-palladium(II) et de dichlorométhane. Le mélange est chauffé à reflux 18 heures, puis versé dans 100 ml d'eau. Le produit est extrait avec du dichlorométhane. La phase organique est séparée, séchée sur sulfate de sodium et le solvant est éliminé par évaporation sous pression réduite. Le solide marron obtenu est alors chromatographié sur colonne de gel de silice greffée aminopropyle (*SiNH2 ;* 30 g) en éluant avec un mélange de dichlorométhane et d'éther de pétrole (70/30) pour donner 0,62 g de 6-chloro-2-(4-fluorophényl)-3-{1-[(4-méthylphényl)sulfonyl]-1*H*-pyrrolo[2,3-*b*]pyridin-4-yl}imidazo[1,2-*b*]pyridazine sous forme de poudre blanche.

PF : 244-246°C

RMN ¹H (CDClo₃) δ : 8,50 (d, 1 H) ; 8,05 (d, 2H) ; 7,95 (d, 1 H) ; 7,55 (d, 1 H) ; 7,4 (m, 3H) ; 7,25 (m, 2H) ; 7,10 (d, 1 H) ; 6,30 (t, 2H) ; 5,95 (d, 1H) ; 2,35 (s, 3H) ppm.

### Etape 3.3. 2-(4-Fluorophényl)-6-[(3R)-3-méthylpipérazin-1-yl]-3-{1-[(4-méthylphényl)sulfonyl]-1H-pyrrolo[2,3-b]pyridin-4-yl}imidazo[1,2-b]pyridazine

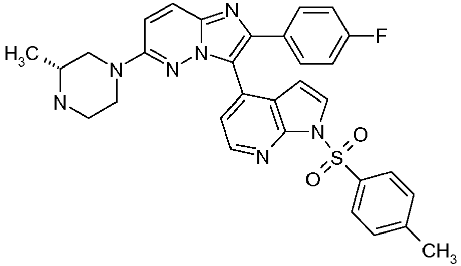

Un mélange de 0,330 g (0,58 mmole) de 6-chloro-2-(4-fluorophényl)-3-{1-[(4-méthylphényl)sulfonyl]-1*H*-pyrrolo[2,3-*b*]pyridin-4-yl}imidazo[1,2-*b*]pyridazine, de 0,116 g (1,16 mmole) de (2*R*)-2-méthylpipérazine et de 0,08 ml (0,6 mmole) de triéthylamine dans 5 ml de pentanol est chauffé à reflux pendant 24 heures. Le milieu réactionnel est dilué avec 100 ml d'une solution aqueuse d'acide chlorhydrique et la solution est lavée avec de l'acétate d'éthyle. La phase aqueuse est alors basifiée par addition d'ammoniaque et le produit est extrait avec du dichlorométhane. La phase organique est séparée, séchée sur sulfate de sodium et le solvant est éliminé par évaporation sous pression réduite. Le solide marron obtenu est alors purifié par chromatographie sur colonne de gel de silice (35 g) en éluant avec un mélange de dichlorométhane, de méthanol et d'ammoniaque (95/5/0,5) pour donner 0,232 g de 2-(4-fluorophényl)-6-[(3*R*)-3-méthylpipérazin-1-yl]-3-{1-[(4-méthylphényl)sulfonyl]-1*H*-pyrrolo[2,3-*b*]pyridin-4-yl}imidazo[1,2-*b*]pyridazine sous forme de poudre jaune après séchage.

PF : 253-256°C

RMN ¹H (CDCl₃) δ : 8,85 (d, 1 H) ; 8,20 (d, 2H) ; 7,85 (d, 1 H) ; 7,65 (d, 1 H) ; 7,5 (m, 3H) ; 7,35 (m, 2H) ; 7,0 (m, 3H) ; 6,20 (d, 1 H) ; 3,9 (m, 2H) ; 3,1 (m, 1 H) ; 2,9 (m, 3H) ; 2,55 (m, 1 H) ; 2,50 (s, 3H) ; 1,8 (sl) ; 1,10 (d, 3H) ppm.

### Etape 3.4. 2-(4-Fluorophényl)-6-[(3R)-3-méthylpipérazin-1-yl]-3-(1H-pyrrolo[2,3-b]pyridin-4-yl)imidazo[1,2-b]pyridazine

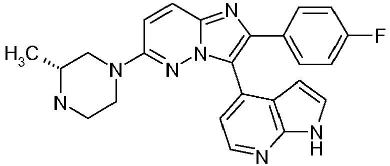

A une solution de 0,230 g (0,40 mmole) de 2-(4-fluorophényl)-6-[(3*R*)-3-méthylpipérazin-1-yl]-3-{1-[(4-méthylphényl)sulfonyl]-1*H*-pyrrolo[2,3-b]pyridin-4-yl}imidazo[1,2-*b*]pyridazine obtenu à l'étape 3.3 dans 5 ml de méthanol, on additionne 0,13 ml (0,76 mmole) d'une solution aqueuse 6N de soude. Le mélange est chauffé à 60°C pendant 30 minutes puis versé dans 100 ml d'eau. Le produit est extrait avec du dichlorométhane. La phase organique est séparée, séchée sur sulfate de sodium et le solvant est éliminé par évaporation sous pression réduite. Le résidu obtenu est recristallisé dans l'acétonitrile pour donner 0,156 g de 2-(4-fluorophényl)-6-[(3*R*)-3-méthylpipérazin-1-yl]-3-(1*H*-pyrrolo[2,3-*b*]pyridin-4-yl)imidazo[1,2-b]pyridazine après séchage.

PF : 285-287°C

[alpha]_{D} = + 4,8° (dichlorométhane, c = 0,998 g/100ml)

RMN ¹H (CDCl₃) δ : 9,3 (sl, 1 H) ; 8,35 (d, 1 H) ; 7,85 (d, 1 H) ; 7,50 (m, 2H) ; 7,30 (d, 1H) ; 7,15 (d, 1 H) ; 6,085 (m, 4H) ; 6,0 (s, 1 H) ; 3,80 (m, 2H) ; 3,45 (s, 1 H) ; 2,95 (s, 1 H) ; 2,80 (m, 3H) ; 2,40 (m, 2H) ; 1,00 (d, 3H) ppm.

### Exemple 4 (composé n°13) : 2-{4-[2-(4-Fluorophényl)-8-méthyl-3-(1H-pyrrolo[2,3-b]pyridin-4-yl)imidazo[1,2-b]pyridazin-6-yl]pipérazin-1-yl}éthanol

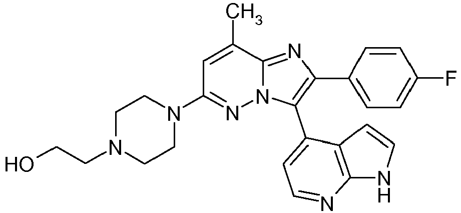

### Etape 4.1. 2-{4-[2-(4-Fluorophényl)-8-méthyl-3-{1-[phénylsulfonyl]-1H-pyrrolo[2,3-b]pyridin-4-yl}imidazo[1,2-b]pyridazin-6-yl]pipérazin-1-yl}éthanol

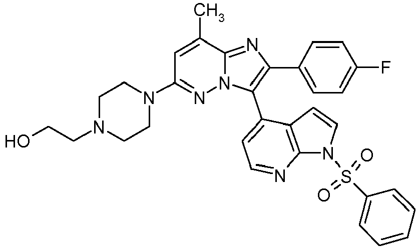

Un mélange de 0,530 g (1,02 mmole) de 6-chloro-2-(4-fluorophényl)-8-méthyl-3-[1-(phénylsulfonyl)-1*H*-pyrrolo[2,3-*b*]pyridin-4-yl]imidazo[1,2-*b*]pyridazine, préparé selon la méthode décrite à l'étape 1.4 de l'exemple 1, de 0,266 g (2,05 mmoles) de 1-(2-hydroxyéthyl)pipérazine (CAS 103-76-4) et de 0,14 ml (1,0 mmole) de triéthylamine dans 5 ml de pentanol est agité pendant 2 jours à 150°C. Le milieu réactionnel est dilué avec 20 ml d'une solution aqueuse d'acide chlorhydrique et la solution est lavée avec de l'acétate d'éthyle. La phase aqueuse est alors basifiée par addition d'ammoniaque et le produit est extrait avec du dichlorométhane. La phase organique est séparée, séchée sur sulfate de sodium et le solvant éliminé par évaporation sous pression réduite. L'huile brune obtenue est alors purifiée par chromatographie sur colonne de gel de silice (40 g) en éluant avec un mélange de dichlorométhane, de méthanol et d'ammoniaque (95/5/0,5) pour donner 0,220 g de 2-{4-[2-(4-fluorophényl)-8-méthyl-3-{1-[phénylsulfonyl]-1*H*-pyrrolo[2,3-*b*]pyridin-4-yl}imidazo[1,2-*b*]pyridazin-6-yl]pipérazin-1-yl}éthanol sous forme de poudre amorphe qui est engagée dans l'étape suivante.

RMN ¹H (CDCl₃) δ : 8,40 (d, 1H) ; 8,15 (d, 2H) ; 7,6-7,3 (m, 7H) ; 6,85 (pt, 2H) ; 6,65 (s, 1 H) ; 6,05 (d, 1 H) ; 3,6 (m, 2H) ; 3,3 (m, 4H) ; 2,6 (s, 3H) ; 2,5 (m, 6H) ppm.

### Etape 4.2. 2-{4-[2-(4-Fluorophényl)-8-méthyl-3-(1H-pyrrolo[2,3-b]pyridin-4-yl)imidazo[1,2-b]pyridazin-6-yl]pipérazin-1-yl}éthanol

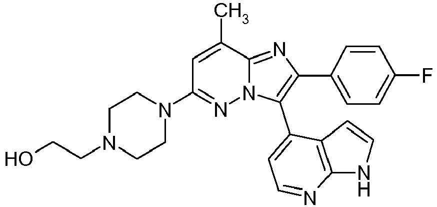

A une solution de 0,22 g (0,36 mmole) de 2-{4-[2-(4-fluorophényl)-8-méthyl-3-{1-[phénylsulfonyl]-1*H*-pyrrolo[2,3-*b*]pyridin-4-yl}imidazo[1,2-*b*]pyridazin-6-yl]pipérazin-1-yl}éthanol dans 5 ml d'un mélange de tétrahydrofurane et de méthanol (1/1), on additionne 0,12 ml (0,72 mmole) d'une solution aqueuse 6N de soude. Le mélange est chauffé à 50°C pendant 1 heure puis versé dans 20 ml d'eau. Le produit est extrait avec du dichlorométhane. La phase organique est séparée, séchée sur sulfate de sodium et le solvant est éliminé par évaporation sous pression réduite. Le résidu jaunâtre obtenu est alors purifié par chromatographie sur colonne de gel de silice (40 g) en éluant avec un mélange de dichlorométhane, de méthanol et d'ammoniaque (95/5/0,5) pour donner 0,110 g de 2-{4-[2-(4-fluorophényl)-8-méthyl-3-(1*H*-pyrrolo[2,3-*b*]pyridin-4-yl)imidazo[1,2-*b*]pyridazin-6-yl]pipérazin-1-yl}éthanol après cristallisation dans 10 ml d'acétonitrile, filtration et séchage.

PF : 239-242°C

RMN ¹H (CDCl₃) δ : 8,35 (d, 1 H) ; 7,55 (2d, 2H) ; 7,40 (d, 1 H) ; 7,30 (d, 1 H) ; 7,20 (s, 1H) ; 7,10 (pt, 2H) ; 5,90 (d, 1 H) ; 4,40 (t, 1 H) ; 3,50 (m, 2H) ; 3,3 (m, 4H) ; 2,60 (s, 3H) ; 2,50 (m, 4H) ; 2,40 (t, 2H) ppm.

### Exemple 5 (composé n°35) : 9-[2-(4-Fluorophényl)-3-(1H-pyrrolo[2,3-b]pyridin-4-yl)imidazo[1,2-b]pyridazin-6-yl]-2,9-diazaspiro[5.5]undécane

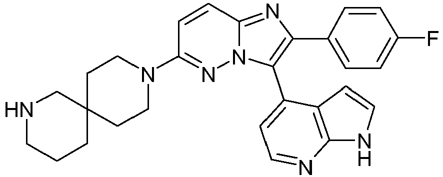

### Etape 5.1. 9-{2-(4-Fluorophényl)-3-[1-(4-méthylphénylsulfonyl)-1H-pyrrolo[2,3-b]pyridin-4-yl]imidazo[1,2-b]pyridazin-6-yl}-2,9-diazaspiro[5.5]undécane-2-carboxylate de tertio-butyle

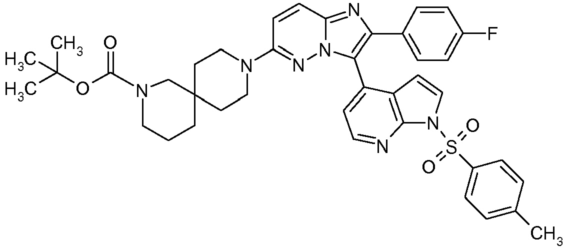

Un mélange de 0,15 g (0,29 mmole) de 6-chloro-2-(4-fluorophényl)-3-[1-(4-méthylphénylsulfonyl)-1*H*-pyrrolo[2,3-*b*]pyridin-4-yl]imidazo[1,2-*b*]pyridazine, préparé selon la méthode décrite à l'étape 3.2 de l'exemple 3, de 0,337 g (1,15 mmole) de chlorhydrate (1:1) de 2,9-diazaspiro[5.5]undécane-2-carboxylate de tertiobutyle (CAS 1023301-88-3) et de 0,224 g (1,7 mmole) de diisopropyléthylamine dans 2 ml de pentanol est chauffé à reflux pendant 40 heures à 140°C. Le solvant est alors évaporé sous pression réduite et le résidu est purifié par chromatographie sur colonne de gel de silice en éluant avec un gradient de dichlorométhane, de méthanol et d'ammoniaque (de 100/0/0 à 90/10/1) pour donner 0,190 mg de 9-{2-(4-fluorophényl)-3-[1-(4-méthylphénylsulfonyl)-1*H*-pyrrolo[2,3-*b*]pyridin-4-yl]imidazo[1,2-*b*]pyridazin-6-yl}-2,9-diazaspiro[5.5]undécane-2-carboxylate de tertio-butyle après cristallisation dans du méthanol.

### Etape 5.2. 9-[2-(4-Fluorophényl)-3-(1H-pyrrolo[2,3-b]pyridin-4-yl)imidazo[1,2-b]pyridazin-6-yl]-2,9-diazaspiro[5.5]undécane-2-carboxylate de tertio-butyle

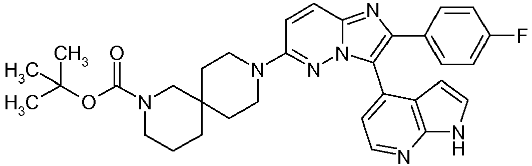

Le 9-{2-(4-fluorophényl)-3-[1-(4-méthylphénylsulfonyl)-1*H*-pyrrolo[2,3-*b*]pyridin-4-yl]imidazo[1,2-*b*]pyridazin-6-yl}-2,9-diazaspiro[5.5]undécane-2-carboxylate de tertio-butyle obtenu à l'étape 5.1 est dissout dans 3 ml d'un mélange de méthanol et de tétrahydrofurane (2/1) et est traité au moyen de 0,09 ml (0,54 mmoles) d'une solution aqueuse de soude 6N à 60 °C pendant 1 heure et demie. Le solvant est évaporé sous pression réduite et le résidu repris dans 3 ml d'eau. Le produit est extrait 2 fois avec 3 ml de dichlorométhane. La phase organique est séchée sur sulfate de sodium et le solvant est éliminé par évaporation sous pression réduite. Le résidu obtenu est alors purifié par chromatographie sur colonne de gel de silice (4 g) en éluant avec un gradient de dichlorométhane, de méthanol et d'ammoniaque (de 95/5/05 à 90/10/1) pour donner 0,06 g de 9-[2-(4-fluorophényl)-3-(1*H*-pyrrolo[2,3-b]pyridin-4-yl)imidazo[1,2-*b*]pyridazin-6-yl]-2,9-diazaspiro[5.5]undécane-2-carboxylate de tertio-butyle après cristallisation dans 10 ml d'acétonitrile, filtration et séchage.

PF : 192-193°C

M+H = 582

RMN ¹H (DMSOd₆) δ : 8,35 (d, 1 H) ; 7,95 (d, 1 H) ; 7,50 (m, 2H) ; 7,40 (d, 1 H) ; 7,30 (m, 2H) ; 7,10 (pt, 2H) ; 5,85 (d, 1H) ; 3,55 (sl) ; 3,40-3,10 (m) ; 1,2-15 (m) ppm.

### Etape 5.3. 9-[2-(4-Fluorophényl)-3-(1H-pyrrolo[2,3-b]pyridin-4-yl)imidazo[1,2-b]pyridazin-6-yl]-2,9-diazaspiro[5.5]undécane

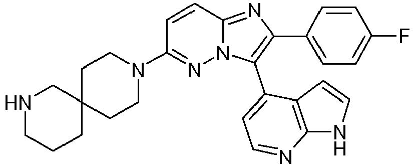

0,20 mg (0,34 mmole) de 9-[2-(4-fluorophényl)-3-(1*H*-pyrrolo[2,3-*b*]pyridin-4-yl)imidazo[1,2-*b*]pyridazin-6-yl]-2,9-diazaspiro[5.5]undécane-2-carboxylate de tertio-butyle sont traités par 5 ml d'acide chlorhydrique 3N aqueux pendant 18 heures à température ambiante. Le milieu réactionnel est versé dans 20 ml d'eau et est neutralisé par addition de soude concentrée. Le produit est ensuite extrait avec du dichlorométhane puis la phase organique est séchée sur sulfate de sodium et le solvant est éliminé par évaporation sous pression réduite.

Le résidu obtenu est alors purifié par chromatographie sur colonne de gel de silice en éluant avec un mélange de dichlorométhane, de méthanol et d'ammoniaque (90/10/1) pour donner 0,07 g de 9-[2-(4-fluorophényl)-3-(1*H*-pyrrolo[2,3-*b*]pyridin-4-yl)imidazo[1,2-*b*]pyridazin-6-yl]-2,9-diazaspiro[5.5]undécane.

PF : 279-280°C

RMN ¹H (DMSOd₆) δ : 11, 7 (s, 1H) ; 8,35 (d, 1 H) ; 7,95 (d, 1H) ; 7,50 (m, 2H) ; 7,40 (d, 1H) ; 7,30 (m, 2H) ; 7,10 (pt, 2H) ; 5,90 (d, 1H) ; 3,4-3,25 (2m, 4H) ; 2,6 (m, 4H) ; 1,6-1,35 (2m, 8H) ppm.

### Exemple 6 (composé n° 36) : 2-(4-Fluorophényl)-6-(4-pyrrolidin-1-ylpipéridin-1-yl)-3-(1H-pyrrolo[2,3-b]pyridin-4-yl)imidazo[1,2-b]pyridazine

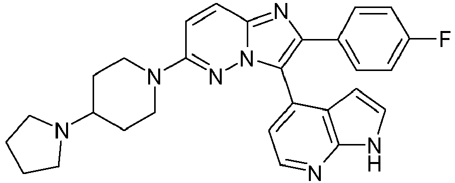

### Etape 6.1. 2-(4-Fluorophényl)-6-(4-pyrrolidin-1-ylpipéridin-1-yl)-3-[1-(4-méthylphénylsulfonyl)-1H-pyrrolo[2,3-b]pyridin-4-yl]imidazo[1,2-b]pyridazine

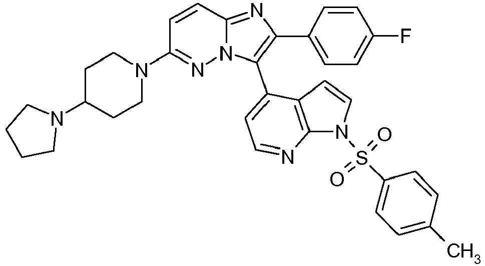

Un mélange de 0,15 g (0,29 mmole) de 6-chloro-2-(4-fluorophényl)-3-[1-(4-méthylphénylsulfonyl)-1*H*-pyrrolo[2,3-*b*]pyridin-4-yl]imidazo[1,2-*b*]pyridazine, préparé selon la méthode décrite à l'étape 3.2 de l'exemple 3, et de 0,179 g (1,16 mmole) de 4-pyrrolidin-1-yl-pipéridine est chauffé à reflux pendant 40 heures à 140°C. Le milieu réactionnel est refroidit. Le solide cristallisé qui se forme au refroidissement est trituré dans 1 ml de d'éther diisopropylique et est isolé par centrifugation et élimination du surnageant pour donner 0,144 g de 2-(4-fluorophényl)-6-(4-pyrrolidin-1-yl-pipéridin-1-yl)-3-[1-(4-méthylphénylsulfonyl)-1*H*-pyrrolo[2,3-*b*]pyridin-4-yl]imidazo[1,2-*b*]pyridazine, utilisé sans purification additionnelle dans la suite de la synthèse.

M+H = 636

Etape 6.2. 2-(4-Fluorophényl)-6-(4-pyrrolidin-1-ylpipéridin-1-yl)-3-(1*H*-pyrrolo[2,3-*b*]pyridin-4-yl)imidazo[1,2-*b*]pyridazine

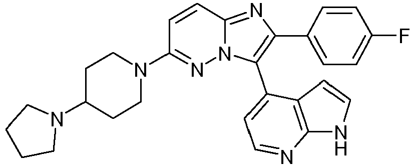

Le 2-(4-fluorophényl)-6-(4-pyrrolidin-1-yl-pipéridin-1-yl)-3-[1-(4-méthylphénylsulfonyl)-1*H-*pyrrolo[2,3-*b*]pyridin-4-yl]imidazo[1,2-*b*]pyridazine obtenu à l'étape 6.1 est dissout dans 3 ml d'un mélange de méthanol et de tétrahydrofurane (2/1), puis traité au moyen de 0,09 ml (0,54 mmoles) d'une solution aqueuse de soude 6N à 60 °C pendant 1 heure et demie. Le solvant est évaporé et le résidu repris dans 3 ml d'eau. Le produit est extrait 2 fois avec 3 ml de dichlorométhane. La phase organique est séchée sur sulfate de sodium et le solvant est éliminé par évaporation sous pression réduite. Le résidu obtenu est alors purifié par chromatographie sur colonne de gel de silice (4 g) en éluant avec un gradient de dichlorométhane, de méthanol et d'ammoniaque (de 95/5/05 à 90/10/1) pour donner 0,064 g de 2-(4-fluorophényl)-6-(4-pyrrolidin-1-ylpipéridin-1-yl)-3-(1*H*-pyrrolo[2,3-*b*]pyridin-4-yl)imidazo[1,2-b]pyridazine après cristallisation dans 10 ml d'acétonitrile, filtration et séchage.

PF : 261-264°C

M+H = 582

RMN ¹H (DMSOd₆) δ : 11, 7 (s, 1 H) ; 8,35 (d, 1 H) ; 7,95 (d, 1 H) ; 7,50 (m, 2H) ; 7,40 (d, 1H) ; 7,30 (m, 2H) ; 7,10 (pt, 2H) ; 5,85 (d, 1 H) ; 2,9 (m, 2H) ; 2,45 (m, 4H) ; 2,15 (m, 1H) ; 1,85 (m, 2H) ; 1,7 (m, 4H), 1,4 (m, 2H) ppm.

### Exemple 7 (composé n° 16) : (R)-1-{4-[2-(4-Fluoro-phényl)-8-méthyl-3-(1H-pyrrolo[2,3-b]pyridin-4-yl)-imidazo[1,2-b]pyridazin-6-yl]-pipérazin-1-yl}-propan-2-ol

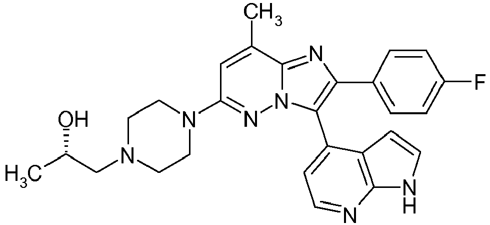

### Etape 7.1. (R)-1-(4-Benzyl-pipérazin-1-yl)-2-hydroxy-propan-1-one

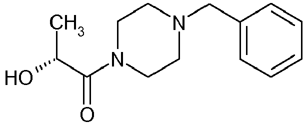

Un mélange de 10,3 g (87,2 mmoles) de (R)-lactate d'éthyle (CAS 7699-00-5) et de 15,3 g de benzylpipérazine (CAS 2759-28-6) est chauffé à 150°C dans un four micro-ondes pendant 2 heures. Le milieu réactionnel est refroidi et est chromatographié sur cartouche de gel de silice en éluant avec un mélange d'acétate d'éthyle et de méthanol (99/1 puis 98/2) pour conduire à 10 g de (*R*)-1-(4-benzyl-pipérazin-1-yl)-2-hydroxy-propan-1-one sous forme d'huile brune.

[alpha]_{D} = +2,4° (méthanol, c = 1g/100ml)

RMN ¹H (DMSO d₆) δ : 7,35 (m, 5H) ; 4,45 (m, 1H) ; 3,85 (m, 1H) ; 3,7 (m, 2H) ; 3,55 (s, 2H) ; 3,45 (m, 2H) ; 2,5 (m, 4H) ; 1,35 (d, 3H) ppm.

### Etape 7.2. (R)-1-(4-Benzyl-pipérazin-1-yl)-propan-2-ol

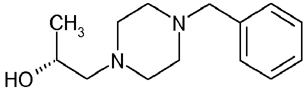

A une suspension de 3,9 g (103 mmoles) d'hydrure de lithium aluminium dans 200 ml de tétrahydrofurane, à 20°C sous agitation on additionne goutte à goutte en 20 minutes 12,8 g (51,7 mmoles) de (R)-1-(4-benzyl-pipérazin-1-yl)-2-hydroxy-propan-1-one en solution dans 100 ml de tétrahydrofurane. On observe une élévation de la température du milieu réactionnel jusqu'à 35°C et on laisse redescendre la température de la réaction jusqu'à température ambiante. Après 30 minutes, l'excès d'hydrure est hydrolysé par addition de sulfate de sodium hydraté puis le mélange est alors filtré et le résidu solide est lavé avec du tétrahydrofurane. Le filtrat est concentré sous pression réduite pour donner 11 g d'une huile jaune qui est chromatographiée sur cartouche de gel de silice en éluant avec un mélange d'acétate d'éthyle, de méthanol et d'ammoniaque (95/5/0,5) pour conduire à 6,4 g de (*R*)-1-(4-benzyl-pipérazin-1-yl)-propan-2-ol sous forme d'huile jaune.

[alpha]_{D} = -20,5° (méthanol, c = 0,1g/100ml)

RMN ¹H (CDCl₃) δ : 7,25 (m, 5H) ; 4,20 (d, 1H) ; 3,70 (m, 1H) ; 3,45 (s, 2H) ; 2,4 et 2,2 (m, 10H) ; 1,0 (d, 3H) ppm.

### Etape 7.3. Dichlorhydrate de (R)-1-(pipérazin-1-yl)-propan-2-ol

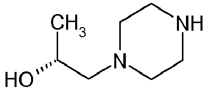

Une solution de 6,2 g (26,5 mmoles) de (*R*)-1-(4-benzyl-pipérazin-1-yl)-propan-2-ol dans 60 ml de méthanol est hydrogénée sous une pression d'hydrogène de 60 psi à température ambiante pendant 2 heures en présence de 2,95 g d'hydroxyde de Palladium sur charbon (CAS 12135-22-7). Le mélange est ensuite filtré sur Büchner et le filtrat est concentré sous pression réduite pour donner 3,8 g d'huile jaune. L'huile est diluée dans environ 60 ml d'isopropanol et la solution est acidifiée par addition d'acide chlorhydrique 5-6 N en solution dans l'isopropanol. Le précipité est agité pendant 15 minutes et est isolé par filtration pour donner après séchage 4,97 g de dichlorhydrate de (*R*)-1-(pipérazin-1-yl)-propan-2-ol sous forme de poudre blanche.

PF : 222-224°C

[alpha]_{D} = -29,2° (méthanol, c = 1g/100ml)

RMN ¹H (CDCl₃) δ : 3,8 (m, 1H) ; 2,9 (m, 3H) ; 2,65 (m, 4H) ; 2,35 et 2,2 (m et m, 3H) ; 1,15 (d, 3H) ppm..

### Etape 7.4. (R)-1-{4-[2-(4-Fluoro-phényl)-8-méthyl-3-(1H-pyrrolo[2,3-b]pyridin-4-yl)-imidazo[1,2-b]pyridazin-6-yl]-pipérazin-1-yl}-propan-2-ol

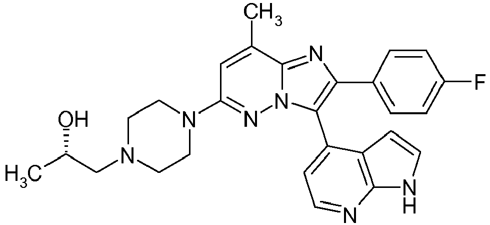

Une solution de 0,450 g (1,19 mmole) de 6-chloro-2-(4-fluorophényl)-8-méthyl-3-(1*H-*pyrrolo[2,3-*b*]pyridin-4-yl)imidazo[1,2-*b*]pyridazine, préparé selon la méthode décrite à l'étape 1.5 de l'exemple 1, de 0,517 g (2,38 mmoles) de dichlorhydrate de (R)-1-(pipérazin-1-yl)-propan-2-ol et de 0,98 ml de diisopropyl-éthyl-amine dans 5 ml de diméthylsulfoxyde est chauffé à 85°C pendant 7 jours. Après refroidissement, le milieu réactionnel est versé dans l'eau et le produit est extrait avec de l'acétate d'éthyle. La phase organique est ensuite séchée sur sulfate de sodium puis concentrée sous pression réduite. Le résidu brun obtenu est alors purifié par chromatographie sur gel de silice en éluant avec un mélange de dichlorométhane, de méthanol et d'ammoniaque (95/5/0,5) pour conduire à 0,04 g de (*R*)-1-{4-[2-(4-fluoro-phényl)-8-méthyl-3-(1*H*-pyrrolo[2,3-*b*]pyridin-4-yl)-imidazo[1,2-*b*]pyridazin-6-yl]-pipérazin-1-yl}-propan-2-ol après recristallisation dans 40 ml d'acétonitrile, filtration et séchage.

PF : >350°C

[alpha]_{D} = -12,6° (méthanol, c = 0,09 g/100ml)

RMN ¹H (DMSO d6) δ : 11,7 (s large, 1 H) ; 8,35 (d, 1 H) ; 7,50 (m, 2H) ; 7,40 (m, 1 H) ; 7,30 (dd, 1 H) ; 7,20 (s, 1H) ; 7,10 (m, 2H) ; 5,85 (m, 1 H) ; 4,30 (m, 1 H) ; 3,80 (m, 1 H) ; 3,35 (m, 4H+H₂O)) ; 2,60 (s, 3H) ; 2,40 (m , 4H+DMSOd₅) ; 2,25 (m, 2H) ; 1,05 (d, 3H).

### Exemple 8 (composé n° 17) : (S)-1-{4-[2-(4-Fluoro-phényl)-8-méthyl-3-(1H-pyrrolo[2,3-b]pyridin-4-yl)-imidazo[1,2-b]pyridazin-6-yl]-pipérazin-1-yl}-propan-2-ol

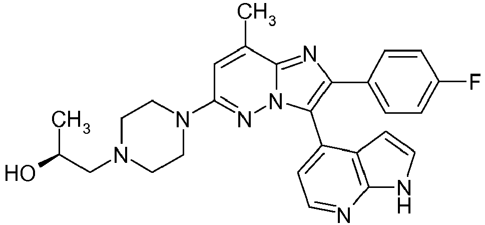

### Etape 8.1. (S)-1-(4-Benzyl-pipérazin-1-yl)-2-hydroxy-propan-1-one

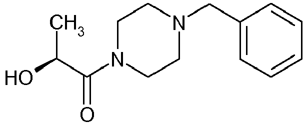

Un mélange de 6,00 g (50,8 mmoles) de (S)-lactate d'éthyle (CAS 687-47-8) et de 9,85 g (50,8 mmoles) de benzylpipérazine (CAS 2759-28-6) est chauffé à 140°C dans un four micro-ondes (300W) pendant 1 heure. Le milieu réactionnel est refroidi puis il est chromatographié sur cartouche de gel de silice en éluant avec un mélange de dichlorométhane, de méthanol et d'ammoniaque (95/5/0,5) pour donner 7g d'huile jaune. Cette huile est diluée dans de l'acétone et le chlorhydrate de (S)-1-(4-benzyl-pipérazin-1-yl)-2-hydroxy-propan-1-one est formé par addition d'une solution d'acide chlorhydrique dans l'isopropanol. Le précité blanc formé est isolé par filtration puis il est repris dans de l'eau et traité au moyen d'ammoniaque. Le produit est alors extrait au moyen de dichlorométhane, la solution séchée sur sulfate de sodium et le solvant évaporé sous pression réduite pour conduire à 3,7 g de (*S*)-1-(4-benzyl-pipérazin-1-yl)-2-hydroxy-propan-1-one sous forme d'huile incolore.

[alpha]_{D} = -2,2° (méthanol, c = 1,56 g/100ml)

RMN ¹H (CDCl₃) δ : 7,25 (m, 5H) ; 4,35 (m, 1H) ; 3,75 (m, 1H) ; 3,6 (m, 2H) ; 3,45 (s, 2H) ; 3,35 (m, 2H) ; 2,4 (m, 4H) ; 1,25 (d, 3H) ppm.

### Etape 8.2. (S)-1-(4-Benzyl-pipérazin-1-yl)-propan-2-ol

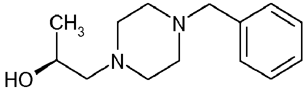

A une suspension de 1,13 g (29,8 mmoles) d'hydrure de lithium-aluminium dans 20 ml de tétrahydrofurane, à 20°C sous agitation on additionne goutte à goutte 3,70 g (14,9 mmoles) de (S)-1-(4-benzyl-pipérazin-1-yl)-2-hydroxy-propan-1-one en solution dans 100 ml de tétrahydrofurane. On laisse redescendre la température de la réaction jusqu'à température ambiante. Après 2 heures, l'excès d'hydrure est hydrolysé par addition de sulfate de sodium hydraté puis le mélange est alors filtré et le filtrat est concentré sous pression réduite L'huile obtenue est chromatographiée sur cartouche de gel de silice en éluant avec un mélange de méthanol et d'ammoniaque dans le dichlorométhane (100/0/0 à 95/5/0,5) pour conduire à 1,2 g de (*S*)-1-(4-benzyl-pipérazin-1-yl)-propan-2-ol sous forme d'huile jaune.

[alpha]_{D} = +23,2° (méthanol, c = 1g/100ml)

RMN ¹H (CDCl₃) δ : 7,3 (m, 5H) ; 3,85 (m, 1H) ; 3,65 (s, 2H) ; 2,8-2,2 (m, 10H) 1,15 (d, 3H) ppm.

### Etape 8.3. (S)-1-(pipérazin-1-yl)-propan-2-ol

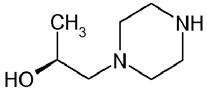

Une solution de 1,2 g (5,1 mmoles) de (*S*)-1-(4-benzyl-pipérazin-1-yl)-propan-2-ol dans 50 ml de méthanol est hydrogénée sous une pression d'hydrogène de 50 psi à température ambiante pendant 2 heures en présence de 0,6 g d'hydroxyde de Palladium. Le mélange est ensuite filtré sur Büchner et le filtrat est concentré sous pression réduite pour donner 0,5 g d'huile jaune.

[alpha]_{D} = +30,5° (méthanol, c = 1g/100ml)

RMN ¹H (CDCl₃) δ : 3,8 (m, 1H) ; 2,8 (m, 4H) ; 2,65 -2,05 (m, 8H) ; 1,05 (d, 3H) ppm.

### Etape 8.4. (S)-1-{4-[2-(4-Fluoro-phényl)-8-méthyl-3-(1H-pyrrolo[2,3-b]pyridin-4-yl)-imidazo[1,2-b]pyridazin-6-yl]-pipérazin-1-yl}-propan-2-ol

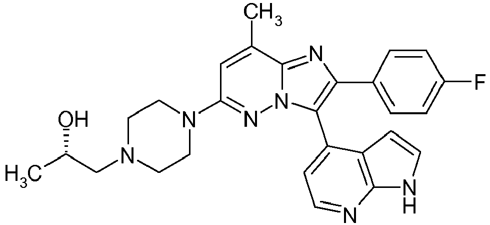

Une solution de 0,300 g (0,79 mmole) de 6-chloro-2-(4-fluorophényl)-8-méthyl-3-(1*H-*pyrrolo[2,3-*b*]pyridin-4-yl)imidazo[1,2-*b*]pyridazine, préparé selon la méthode décrite à l'étape 1.5 de l'exemple 1, de 0,345 g (1,59 mmole) de (*S*)-1-(pipérazin-1-yl)-propan-2-ol et de 0,45 ml (3,18 mmoles) de diisopropyl-éthyl-amine dans 5 ml de pentanol est chauffée à 150°C pendant 8 jours. Après refroidissement, le milieu réactionnel est versé dans une solution aqueuse 1 N d'acide chlorhydrique et la phase aqueuse est lavée avec de l'acétate d'éthyle. La phase aqueuse est alors basifiée au moyen d'une solution aqueuse d'ammoniaque et le produit est extrait avec du dichlorométhane. La phase organique est ensuite séchée sur sulfate de sodium puis concentrée sous pression réduite. Le résidu brun obtenu est alors purifié par chromatographie sur cartouche de gel de silice en éluant avec un mélange de dichlorométhane, de méthanol et d'ammoniaque (95/5/0,5) pour conduire à 0,05 g de (*S*)-1-{4-[2-(4-fluoro-phényl)-8-méthyl-3-(1*H*-pyrrolo[2,3-*b*]pyridin-4-yl)-imidazo[1,2-*b*]pyridazin-6-yl]-pipérazin-1-yl}-propan-2-ol après recristallisation dans l'acétonitrile, filtration et séchage.

PF : >350°C

[Alpha]_{D} = +13,9° (méthanol, c = 0,2 g/100ml)

RMN ¹H (DMSO d6) δ : 11,7 (s large, 1 H) ; 8,35 (d, 1 H) ; 7,50 (m, 2H) ; 7,40 (m, 1 H) ; 7,30 (dd, 1 H) ; 7,20 (s, 1 H) ; 7,10 (m, 2H) ; 5,85 (m, 1 H) ; 4,30 (m, 1 H) ; 3,80 (m, 1 H) ; 3,35 (m, 4H) ; 2,60 (s, 3H) ; 2,40 (m , 4H) ; 2,25 (m, 2H) ; 1,05 (d, 3H).

Le tableau 1 qui suit illustre les structures chimiques et les propriétés physiques de quelques composés selon l'invention.

Dans ce tableau :
- dans la colonne « Sel », « - » représente un composé sous forme de base libre, alors que « HCl » représente un composé sous forme de chlorhydrate et le rapport entre parenthèses est le rapport (acide:base) ;
- la colonne "PF°C" renseigne les points de fusion des produits en degrés Celsius. "N.D" signifie que le point de fusion est non déterminé,
- la colonne [α]*_{D}* renseigne le résultat d'analyse du pouvoir rotatoire des composés du tableau à la longueur d'onde de 589 nM ; le solvant indiqué entre parenthèses correspond au solvant employé pour réaliser la mesure du pouvoir rotatoire en degrés et la lettre « c » indique la concentration du solvant en g/100 ml. « N.A. » signifie que la mesure du pouvoir rotatoire n'est pas applicable,
- la colonne "m/z" renseigne l'ion moléculaire (M+H⁺) ou (M⁺) observé par analyse des produits par spectrométrie de masse, soit par LC-MS (liquid chromatography coupled to Mass Spectroscopy) réalisée sur un appareil de type Agilent LC-MSD Trap en mode ESI positif, soit par introduction directe par MS (Mass Spectroscopy) sur un appareil Autospec M (EBE) en utilisant la technique DCI-NH3 ou en utilisant la technique d'impact électronique sur un appareil de type Waters GCT. Les valeurs ayant un astérisque « * » correspondent à la détection de l'ion (M⁺).
- « CH₃- » signifie méthyle,
- « CH₃OH » signifie méthanol,
- « CH₂Cl₂ » signifie dichlorométhane,
- « DMSO » signifie diméthylsulfoxyde.

**Tableau 1**

| | | | | | | | | |
|---|---|---|---|---|---|---|---|---|
| 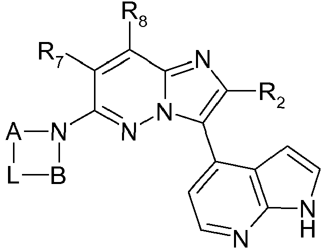 | | | | | | | | |

| **No** | **NALB** | **R₇** | **R₈** | **R₂** | **Sel** | **m/z** | **[α]*_{D}* (°)** (c en g/100ml ; solvant) | ***PF°C*** |
|---|---|---|---|---|---|---|---|---|
| 1 | (*R*)-3-Méthyl-pipérazin-1-yle | H | H | CH₃- | - | 348 | + 29 (c=0,683 ; CH₃OH) | 202-204 |
| 2 | 3,3-Diméthyl-pipérazin-1-yle | H | H | CH₃- | HCl (3:1) | 362 | N.A. | 220 décomposition |
| 3 | (*cis*)-3,5-Diméthyl-pipérazin-1-yle | H | H | CH₃- | HCl (3:1) | 362 | N.A | 235 décomposition |
| 4 | 4-Isopropyl-pipérazin-1-yle | H | H | CH₃- | HCl (3:1) | 376 | N.A. | 195 |
| 5 | (*cis*)-5-Méthyl-hexahydro-pyrrolo[3,4-c]pyrrol-2(1 *H*)-yle | H | H | CH₃- | HCl (3:1) | *373 | N.A. | 205 décomposition |
| 6 | (*R*)-3-Méthyl-pipérazin-1-yle | H | H | 4-F-Phényl | - | 428 | + 4,8 (c=0,998 ; CH₂Cl₂) | 285-287 |
| 7 | 3-Hydroxyméthyl-pipérazin-1-yle | H | H | 4-F-Phényl | - | 444 | N.A | 234-238 |
| 8 | 3,3-Diméthyl-pipérazin-1-yle | H | H | 4-F-Phényl | - | 442 | N.A. | 284-287 |
| 9 | 3,3-Diméthyl-pipérazin-1-yle | H | CH₃ | 3-F-Phényl | - | 456 | N.A. | 270-273 |
| 10 | 3,3-Diméthyl-pipérazin-1-yle | H | CH₃ | 4-F-Phényl | - | 456 | N.A. | > 300 |
| 11 | (cis)-3,5-Diméthyl-pipérazin-1-yle | H | H | 4-F-Phényl | - | 442 | N.A. | 288-290 |
| 12 | 4-(2-Hydroxy-éthyl)-pipérazin-1-yle | H | CH₃ | 3-F-Phényl | - | 472 | N.A. | 201-203 |
| 13 | 4-(2-Hydroxy-éthyl)-pipérazin-1-yle | H | CH₃ | 4-F-Phényl | - | 472 | N.A. | 239-242 (butanol) 187-198 (acétonitrile) |
| 14 | 4-Isopropyl-pipérazin-1-yle | H | H | 4-F-Phényl | - | 456 | N.A. | 271-273 |
| 15 | 4-Isopropyl-pipérazin-1-yle | H | CH₃ | 4-F-Phényl | - | 470 | N.A. | 285-291 |
| 16 | (*R*)-4-(2-Hydroxy-propyl)-pipérazin-1-yle | H | CH₃ | 4-F-Phényl | - | 486 | -12,6 (c=0,09 ; CH30H) | >350°C |
| 17 | (*S*)-4-(2-Hydroxy-propyl)-pipérazin-1-yle | H | CH₃ | 4-F-Phényl | - | 486 | +13,9 (c = 0,2 ; CH3OH) | >350°C |
| 18 | 6,9-Diaza-spiro[4.5]déc-9-yle | H | CH₃ | 4-F-Phényl | - | 482 | N.A. | 293-299 |
| 19 | 4-(1-hydroxy-2-méthyl-propan-2-yl)-pipérazin-1-yle | H | H | 4-F-Phényl | - | 486 | N.A. | >250°C |
| 20 | 4-(2-Hydroxy-2-méthyl-propyl)-pipérazin-1-yle | H | H | 4-F-Phényl | - | 486 | N.A. | 273-276 |
| 21 | 4-(2-Hydroxy-2-méthyl-propyl)-pipérazin-1-yle | H | CH₃ | 3-F-Phényl | - | 500 | N.A. | >270°C |
| 22 | 4-(2-Hydroxy-2-méthyl-propyl)-pipérazin-1-yle | H | CH₃ | 4-F-Phényl | - | 500 | N.A. | 265-268 |
| 23 | 4-(3-Hydroxy-3-méthyl-butyl)-pipérazin-1-yle | H | H | 4-F-Phényl | - | 500 | N.A. | 73-75 |
| 24 | (*R*)-3-Phényl-pipérazin-1-yle | H | H | 4-F-Phényl | HCl (3:1) | 490 | -27 (c=0,714 ; CH₃OH) | 215°C décomposition |
| 25 | (*S*)-3-Phényl-pipérazin-1-yle | H | H | 4-F-Phényl | HCl (3:1) | 490 | +22 (c=0,622 ; CH₃OH) | 215°C décomposition |
| 26 | 3-Phényl-pipérazin-1-yle | H | CH₃ | 4-F-Phényl | - | 504 | N.A. | 253-257 |
| 27 | 4-Benzyl-pipérazin-1-yle | H | H | 4-F-Phényl | - | 504 | N.A. | 274-278 |
| 28 | (*cis*)-5-Méthyl-hexahydro-pyrrolo[3,4-c]pyrrol-2(1*H*)-yle | H | H | 4-F-Phényl | - | 454 | N.A. | 238-239 |
| 29 | (*cis*)-5-Méthyl-hexahydro-pyrrolo[3,4-c]pyrrol-2(1*H*)-yle | H | CH₃ | 4-F-Phényl | - | 468 | N.A. | 255 décomposition |
| 30 | (*cis*)-5-(2-Hydroxy-éthyl)-hexahydro-pyrrolo[3,4-*c*]pyrrol-2(1*H*)-yle | H | H | 4-F-Phényl | - | 484 | N.A. | 175-180 |
| 31 | (*cis*)-5-(2-Hydroxy-éthyl)-hexahydro-pyrrolo[3,4-*c*]pyrrol-2(1*H*)-yle | H | CH₃ | 4-F-Phényl | - | 498 | N.A. | 271-275 |
| 32 | (*4aR*, *7aR*)-1-méthyl-octahydro-pyrrolo[3,4-*b*]pyridin-6-yle | H | CH₃ | 4-F-Phényl | - | 482 | +24,4 (c=0,492 ; CH₃OH) | >260°C |
| 33 | (*4aS, 7aS*)-1-méthyl-octahydro-pyrrolo[3,4-*b*]pyridin-6-yle | H | CH₃ | 4-F-Phényl | - | 482 | -21,8 (c=0,478 ; CH₃OH) | >260°C |
| 34 | (1 *S*,4S)-5-Méthyl-2,5-diaza-bicyclo[2.2.1]hept-2-yle | H | H | 4-F-Phényl | - | 440 | -66,0 (c= 0,961 ; DMSO) | 148-168 |
| 35 | 2,9-Diaza-spiro[5.5]undéc-9-yle | H | H | 4-F-Phényl | - | 482 | N.A. | 279-280 |
| 36 | 4-(Pyrrolidin-1-yl)-pipéridin-1-yle | H | H | 4-F-Phényl | - | 482 | N.A. | 261-264 |

### Exemples biologiques

La capacité des composés de l'invention à inhiber la phosphorylation de la caséine par les caséines kinase 1 epsilon et delta peut être évaluée selon la procédure décrite dans le document US20050131012.

### Dosage sur Plaque-Filtre-d'ATP-³³P pour le criblage des inhibiteurs de CK1epsilon :

On mesure l'effet des composés pour inhiber la phosphorylation de la caséine par l'enzyme caséine kinase 1 epsilon (CK1 epsilon) en utilisant un dosage de la caséine par filtration d'ATP-³³P in vitro.

La Caséine Kinase 1 epsilon (0,58 mg/ml) est obtenue par des procédés de fermentation et de purification effectués selon des méthodes bien connues de l'homme du métier ou peut également être obtenue auprès d'Invitrogen Corporation™ (human CK1 epsilon).

Les composés sont testés à cinq concentrations différentes de manière à générer des Cl₅₀, c'est à dire la concentration à laquelle un composé est capable d'inhiber l'activité enzymatique de 50%, ou bien l'inhibition en % à une concentration de 10 micromolaires.

On prépare des plaques Falcon à fond en « U » en plaçant 5 µL de solutions des composés selon l'invention aux concentrations de 10, 1, 0,1, 0,01 ou 0,001 µM dans différents puits. Les solutions des composés selon l'invention à ces différentes concentrations sont préparées par dilution dans un tampon d'essai (Tris 50 mM pH 7,5, MgCl2 10 M, DTT 2 mM et EGTA 1 mM) d'une solution mère dans le DMSO à la concentration de 10 mM. Ensuite, on additionne 5 µL de caséine déphosphorylée à la concentration finale de 0,2 µg/µL, 20 µL de CK1 epsilon à la concentration finale de 3 ng/µL, et 20 µL d'ATP-³³P à la concentration finale de 0,02 µCi/µL mélangée avec de l'ATP froide (10 µM final - environ 2x106 CPM par puits). Le volume total final d'essai par puits est égal à 50 µL.

La plaque d'essai Falcon^{®} à fond en « U » citée ci-dessus est agitée au vortex, puis incubée à la température ambiante pendant 2 heures. Après 2 heures, la réaction est arrêtée par addition d'une solution glacée de 65 µL d'ATP froid (2 mM) préparée dans du tampon d'essai.

On transfère ensuite 100 µL du mélange réactionnel de la plaque Falcon^{®} à fond en U dans des plaques de filtration MAPH Millipore^{®}, préalablement imprégnées avec 25 µL de TCA glacé à 100 %

Les plaques de filtration MAPH Millipore sont agitées doucement et on les laisse au repos à la température ambiante pendant au moins 30 minutes pour précipiter les protéines.

Après 30 minutes, les plaques de filtration sont séquentiellement lavées et filtrées avec 2x150 µL de TCA à 20%, 2x150 µL de TCA à 10% et 2x150 µL de TCA à 5% (6 lavages au total par plaque/900 µL par puits).

On laisse les plaques sécher pendant une nuit à la température ambiante. Ensuite, on ajoute 40 µL de liquide de scintillation Microscint-20 Packard^{®} par puits et les plaques sont fermées de manière étanche. On mesure alors le rayonnement émis par chaque puits pendant 2 minutes dans un compteur à scintillation Topcount NXT Packard^{®} où les valeurs de CPM /puits sont mesurées.

On détermine l'inhibition en % de la capacité de l'enzyme à phosphoryler le substrat (caséine) pour chaque concentration de composé testé. Ces données d'inhibition exprimées en % sont utilisées pour calculer la valeur de Cl₅₀ pour chaque composé comparativement aux contrôles.

Les études cinétiques ont déterminé la valeur de K_{M} pour ATP comme étant de 21 µM dans ce système d'essai.

Le tableau 2 ci-dessous présente les Cl₅₀ d'inhibition de la phosphorylation de la Caséine Kinase 1 Epsilon pour quelques composés selon l'invention.

**Tableau 2**

| Composé N° | CK1 epsilon Cl₅₀ (nM) |
|---|---|
| 3 | 303 |
| 6 | 1-2 |
| 35 | 6 |
| 36 | 5-8 |

Dans ces conditions, les composés les plus actifs de l'invention présentent des Cl₅₀ (concentration inhibant de 50 % l'activité enzymatique de la Caséine Kinase 1 Epsilon) comprises entre 1 nM et 2 µM.

La capacité des composés de l'invention à inhiber la phosphorylation de la caséine par les caséines kinases 1 epsilon et delta peut être évaluée en utilisant un test de fluorescence FRET (« transfert d'énergie entre molécules fluorescentes », de l'anglais « Fluorescence Resonance Energy Transfert ») à partir du kit « Z'Lyte™ kinase assay Kit » (référence PV3670 ; Invitrogen Corporation™) selon les instructions du fournisseur.

Les Caséines Kinases 1 utilisées sont obtenues chez Invitrogen Corporation (human CK1 epsilon PV3500 et human CK1 delta PV3665).

Un peptide substrat, marqué à ses deux extrémités par un groupe fluorophore donneur (la coumarine) et un groupe fluorophore accepteur (la fluorescéine) constituant un système FRET est phosphorylé en présence d'ATP par la caséine kinases 1 epsilon ou delta en présence de concentrations croissantes de composés de l'invention.

Le mélange est traité au moyen d'une protéase site spécifique coupant spécifiquement le peptide substrat pour former deux fragments fluorescents présentant un grand ratio d'émission par fluorescence.

La fluorescence observée est donc reliée à la capacité des produits de l'invention à inhiber la phosphorylation du peptide substrat par la caséine kinase 1 epsilon ou de la caséine kinase 1 delta.

Les composés de l'invention sont mis en solution à des concentrations différentes à partir d'une solution mère à 10 mM dans le DMSO diluée dans un tampon contenant 50 mM HEPS, pH 7,5, 1 mMEGTA, 0,01% Brij-35, 10 mM MgCl₂ pour la caséine kinase 1 epsilon et supplémenté avec Trizma Base (50 mM), pH 8,0 et NaN3 (0,01% finaux) pour la caséine kinase 1 delta.

La phosphorylation du peptide substrat SER/THR 11 obtenu chez Invitrogen Corporation™ est réalisée à la concentration finale de 2 µM. La concentration en ATP est de 4 fois le K_{M}, celui-ci étant de 2 µM pour la caséine kinase 1 epsilon et de 4 µM pour la caséine kinase 1 delta.

La mesure de la fluorescence émise est réalisée aux longueurs d'onde de 445 et 520 nm (excitation à 400 nm).

Le tableau 3 ci-dessous présente les Cl₅₀ d'inhibition de la phosphorylation de la Caséine Kinase 1 Delta pour quelques composés selon l'invention.

**Tableau 3**

| Composé N° | CK1 delta Cl₅₀ (nM) |
|---|---|
| 20 | 30-42 |
| 29 | 5 |
| 36 | 19 |

Dans ces conditions, les composés les plus actifs de l'invention présentent des Cl₅₀ (concentration inhibant de 50 % l'activité enzymatique de la Caséine Kinase 1 Delta) comprises entre 1 nM et 2 µM.

Il apparaît donc que les composés selon l'invention ont une activité inhibitrice sur l'enzyme Caséine Kinase 1 epsilon ou Caséine Kinase 1 delta.

### Protocoles expérimentaux de dosage circadien cellulaire

Des cultures de fibroblastes Mper1-luc Rat-1 (P2C4) ont été réalisées en divisant les cultures tous les 3-4 jours (environ 10-20 % de confluence) sur des flacons de culture de tissus en polystyrène dégazés de 150 cm² (Falcon® # 35-5001) et maintenues en milieu de croissance [EMEM (Cellgro #10-010-CV) ; sérum bovin foetal à 10 % (FBS; Gibco #16000-044) ; et 50 I.U./mL de pénicilline-streptomycine (Cellgro #30-001-Cl)] à 37°C et sous CO₂ 5 %.

Des cellules issues de cultures de fibroblastes Rat-1 à 30-50 % de confluence telle que décrite ci-dessus ont été co-transfectées avec des vecteurs contenant le marqueur de sélection pour la résistance à la Zéocine pour une transfection stable et un gène rapporteur de la luciférase dirigé par le promoteur mPer-1. Après 24 à 48 heures, les cultures ont été divisées sur des plaques de 96 puits et maintenues en milieu de croissance additionné de 50-100 µg/mL de Zéocine (Invitrogen^{®} #45-0430) pendant 10-14 jours. Les transfectants stables résistant à la Zéocine ont été évalués pour l'expression du rapporteur en ajoutant au milieu de croissance de la luciférine 100 µM (Promega^{®} #E1603^{®}) et en dosant l'activité de la luciférase sur un compteur à scintillation TopCount^{®} (Packard Modèle #C384V00). Les clones de cellule Rat-1 exprimant aussi bien la résistance à la Zéocine que l'activité de la luciférase dirigée par mPer1 ont été synchronisés par choc au sérum avec du sérum de cheval à 50 % [HS (Gibco^{®} #16050-122)] et l'activité du rapporteur circadien a été évaluée. Le clone P2C4 de fibroblastes Mper1-luc Rat-1 a été sélectionné pour l'essai du composé.

Des fibroblastes Mper1-luc Rat-1 (P2C4) à 40-50 % de confluence obtenus selon le protocole décrit précédemment ont été étalés sur des plaques de culture de tissu opaques de 96 puits (Perkin Elmer^{®} #6005680). Les cultures sont maintenues en milieu de croissance additionné de 100 µg/mL de Zéocine (Invitrogen #45-0430) jusqu'à ce qu'elles aient atteint 100 % de confluence (48-72 h). Les cultures ont ensuite été synchronisées avec 100 µL de milieu de synchronisation [EMEM (Cellgro #10-010-CV) ; 100 I.U. /mL de pénicilline-streptomycine (Cellgro #30-001-C1) ; HS à 50% (Gibco #16050-122)] pendant 2 heures à 37°C et sous CO₂ 5%. Après synchronisation, les cultures ont été rincées avec 100 µL d'EMEM (Cellgro #10-010-CV) pendant 10 minutes à température ambiante. Après rinçage, le milieu a été remplacé par 300 µL de milieu indépendant de CO₂ [CO₂I (Gibco #18045-088) ; L-glutamine 2 mM (Cellgro #25-005-C1) ; 100 U.I./mL de pénicilline-streptomycine (Cellgro #30-001-C1) ; luciférine 100 µM (Promega #E 1603)]. Les composés de l'invention testés pour les effets circadiens ont été ajoutés à du milieu indépendant de CO₂ dans du DMSO à 0,3 % (concentration finale). Les cultures ont été fermées immédiatement de manière étanche avec du film TopSeal-A^{®} (Packard #6005185) et transférées pour la mesure de l'activité de luciférase.

Après synchronisation, les plaques d'essai ont été maintenues à 37°C dans une étuve de culture de tissu (Forma Scientific Modèle #3914). L'activité de luciférase In Vivo a été estimée en mesurant l'émission relative de lumière sur un compteur à scintillation TopCount (Packard Modèle #C384V00).

L'analyse de périodes a été effectuée soit en déterminant l'intervalle entre les minimums d'émission relative de lumière sur plusieurs jours ou par transformation de Fourier. Les deux méthodes ont produit une estimation de période pratiquement identique sur une gamme de périodes circadiennes. La puissance est rapportée en CE Delta (t+1 h), qui est présentée comme la concentration micromolaire efficace qui a induit un prolongement de la période de 1 heure. Les données ont été analysées par ajustement d'une courbe hyperbolique aux données exprimées en changement de période (ordonnée) en fonction de la concentration du composé à tester (abscisse) dans le logiciel XLfit™ et la CE Delta (t+1 h) a été interpolée à partir de cette courbe.

Le tableau 4 ci-dessous présente les CE Delta (t+1 h) pour quelques composés selon l'invention.

**Tableau 4**

| Composé N° | CE Delta (t+1h) (nM) |
|---|---|
| 6 | 2-3 |
| 35 | 305 |
| 36 | 1-7 |

Dans ces conditions, les composés les plus actifs de l'invention présentent des CE Delta (t+1 h) (concentration micromolaire efficace qui a induit un prolongement de la période de 1 heure) comprises entre 1 nM et 2 µM.

En inhibant les enzymes CK1 epsilon et/ou de CK1delta, les composés objets de l'invention modulent la rythmicité circadienne, et peuvent être utiles pour le traitement des désordres liés au rythme circadien.

Les composés selon l'invention peuvent notamment être utilisés pour la préparation d'un médicament destiné à prévenir ou à traiter les désordres du sommeil ; les troubles du rythme circadien, tels que notamment ceux dus au décalage horaire, au travail posté. Parmi les troubles du sommeil, on distingue notamment les troubles primaires du sommeil tels que la dyssomnie (par exemple l'insomnie primaire), la parasomnie, l'hypersomnie (par exemple la somnolence excessive), la narcolepsie, les troubles du sommeil liés à l'apnée du sommeil, les troubles du sommeil liés au rythme circadien et les dyssomnies non spécifiées par ailleurs, les troubles du sommeil associés à des troubles médicaux/psychiatriques.

Les composés objets de l'invention provoquent également un déplacement de la phase circadienne et une telle propriété peut être utile dans le cadre d'une monothérapie ou une thérapie combinée potentielle cliniquement efficace pour les troubles de l'humeur.

Parmi les troubles de l'humeur, on distingue notamment les troubles dépressifs (dépression unipolaire), les troubles bipolaires, les troubles de l'humeur dus à une affection médicale générale ainsi que les troubles de l'humeur induits par des substances pharmacologiques. Parmi les troubles bipolaires, on distingue notamment les troubles bipolaires I et troubles bipolaires II, dont notamment les troubles affectifs saisonniers.

Les composés objets de l'invention modulant la rythmicité circadienne, peuvent être utiles dans le traitement des troubles anxieux et dépressifs dus en particulier à une altération sur la sécrétion de CRF.

Parmi les troubles dépressifs, on distingue notamment les troubles dépressifs majeurs, troubles dysthymiques, les troubles dépressifs non spécifiés par ailleurs.

Les composés objets de l'invention modulant la rythmicité circadienne, peuvent être utiles pour la préparation d'un médicament destiné à traiter les maladies liées à la dépendance à des substances d'abus telles que la cocaïne, la morphine, la nicotine, l'éthanol, le cannabis.

En inhibant la caséine kinase 1 epsilon et/ou la caséine kinase 1 delta, les composés selon l'invention peuvent être utilisés pour la préparation de médicaments, notamment pour la préparation d'un médicament destiné à prévenir ou à traiter des maladies reliées à l'hyperphosphorylation de la protéine tau, notamment la maladie d'Alzheimer.

Ces médicaments trouvent également leur emploi en thérapeutique, notamment dans le traitement ou la prévention des maladies causées ou exacerbées par la prolifération des cellules et en particulier des cellules tumorales.

Comme inhibiteur de la prolifération des cellules tumorales, ces composés sont utiles dans la prévention et le traitement des tumeurs liquides telles que les leucémies, des tumeurs solides à la fois primaires et métastasiques, des carcinomes et cancers, en particulier : cancer du sein ; cancer du poumon ; cancer de l'intestin grêle, cancer du colon et du rectum ; cancer des voies respiratoires, de l'oropharynx et de l'hypopharynx ; cancer de l'oesophage ; cancer du foie, cancer de l'estomac, cancer des canaux biliaires, cancer de la vésicule biliaire, cancer du pancréas ; cancers des voies urinaires y compris rein, urothélium et vessie ; cancers du tractus génital féminin y compris cancer de l'utérus, du col de l'utérus, des ovaires, chloriocarcinome et trophoblastome ; cancers du tractus génital masculin y compris cancer de la prostate, des vésicules séminales, des testicules, tumeurs des cellules germinales; cancers des glandes endocrines y compris cancer de la thyroïde, de l'hypophyse, des glandes surrénales ; cancers de la peau y compris hémangiomes, mélanomes, sarcomes, incluant le sarcome de Kaposi ; tumeurs du cerveau, des nerfs, des yeux, des méninges, incluant astrocytomes, gliomes, glioblastomes, rétinoblastomes, neurinomes, neuroblastomes, schwannomes, méningiomes ; tumeurs malignes hématopoïétiques ; leucémies, (Acute Lymphocytic Leukemia (ALL), Acute Myeloid Leukemia (AML), Chronic Myeloid Leukemia (CML), Chronic lymphocytic leukemia (CLL)) chloromes, plasmocytomes, leucémies des cellules T ou B, lymphomes non hodgkiniens ou hodgkiniens, myélomes, hémopathies malignes diverses.

Les composés selon l'invention peuvent également être utilisés pour la préparation de médicaments, notamment pour la préparation d'un médicament destiné à prévenir ou à traiter les maladies inflammatoires, telles que notamment les maladies inflammatoires du système nerveux central comme la sclérose en plaque, encéphalite, myélite et encéphalomyélite et autres maladies inflammatoires comme les pathologies vasculaires, l'athérosclérose, les inflammations des articulations, l'arthrose, l'arthrite rhumatoïde.

Les composés selon l'invention peuvent donc être utilisés pour la préparation de médicaments, en particulier de médicaments inhibiteurs de la caséine kinase 1 epsilon et/ou de la caséine kinase 1 delta.

Ainsi, selon un autre de ses aspects, l'invention a pour objet des médicaments qui comprennent un composé de formule (I), ou un sel d'addition de ce dernier à un acide pharmaceutiquement acceptable ou encore un hydrate ou un solvate du composé de formule (I).

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention ou un sel pharmaceutiquement acceptable, un hydrate ou solvate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intraveineuse, topique, locale, intra trachéale, intra nasale, transdermique ou rectale, le principe actif de formule (I) ci-dessus, ou son sel, solvate ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intra trachéale, intraoculaire, intra nasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Par voie orale, la dose de principe actif administrée par jour peut atteindre 0,1 à 20 mg/kg, en une ou plusieurs prises.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvates.

## Revendications

1. Composé de formule générale (I) 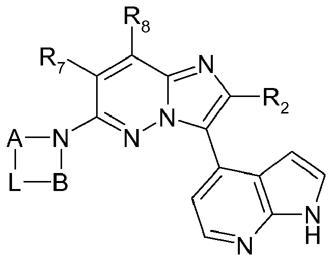 dans laquelle
- R₂ représente un groupe aryle éventuellement substitué par un ou plusieurs substituants choisis parmi les atomes d'halogène et les groupes C₁₋₆-alkyle, C₁₋₆-alkyloxy, C₁₋₆-alkylthio, C₁₋₆-fluoroalkyle, C₁₋₆-fluoroalkyloxy, -CN ou R₂ représente un groupe C₁₋₆-alkyle, C₁₋₆-fluoroalkyle, C₃₋₇-cycloalkyle, C₃₋₇-cycloalkyl-C₁₋₆-alkyle ;
- A représente un groupe C₁₋₇-alkylène éventuellement substitué par un ou deux groupes Rₐ;
- B représente un groupe C₁₋₇-alkylène éventuellement substitué par un groupe R_{b} ;
- L représente, soit un atome d'azote éventuellement substitué par un groupe R_{c} ou R_{d}, soit un atome de carbone substitué par un groupe Rₑ₁ et un groupe R_{d} ou deux groupes Rₑ₂ ; les atomes de carbone de A et de B étant éventuellement substitués par un ou plusieurs groupes R_{f} identiques ou différents l'un de l'autre ;
- Rₐ, R_{b} et R_{c} sont définis tels que :
deux groupes Rₐ peuvent former ensemble un groupe C₁₋₆-alkylène ;
Rₐ et R_{b} peuvent former ensemble une liaison ou un groupe C₁₋₆-alkylène ;
Rₐ et R_{c} peuvent former ensemble une liaison ou un groupe C₁₋₆-alkylène
R_{b} et R_{c} peuvent former ensemble une liaison ou un groupe C₁₋₆-alkylène ;
- R_{d} représente un groupe choisi parmi l'atome d'hydrogène et les groupes C₁₋₆-alkyle, C₃₋₇-cycloalkyle, C₃₋₇-cycloalkyl-C₁₋₆-alkyle, hydroxy-C₁₋₆-alkyle, C₁₋₆-alkyloxy-C₁₋₆-alkyle, C₁₋₆-alkylthio-C₁₋₆-alkyle, C₁₋₆-fluoroalkyle ou benzyle ;
- Rₑ₁ représente un groupe -NR₄R₅ ou une monoamine cyclique comportant éventuellement un atome d'oxygène, la monoamine cyclique étant éventuellement substituée par un ou plusieurs substituants choisis parmi l'atome de fluor et les groupes C₁₋₆-alkyle, C₁₋₆-alkyloxy, hydroxyle ;
- Deux Rₑ₂ forment avec l'atome de carbone qui les porte une monoamine cyclique comportant éventuellement un atome d'oxygène, la monoamine cyclique étant éventuellement substituée par un ou plusieurs groupes R_{f} identiques ou différents l'un de l'autre ;
- R_{f} représente un groupe C₁₋₆-alkyle, C₃₋₇-cycloalkyle, C₃₋₇-cycloalkyle-C₁₋₆-alkyle, C₁₋₆-alkyloxy-C₁₋₆-alkyle, hydroxy-C₁₋₆-alkyle, C₁₋₆-fluoroalkyle, phényle ou benzyle ;
- R₄ et R₅ représentent, indépendamment l'un de l'autre, un atome d'hydrogène ou un groupe C₁₋₆-alkyle, C₃₋₇-cycloalkyle, C₃₋₇-cycloalkyle-C₁₋₆-alkyle ;
- R₇ et R₈ représentent, indépendamment l'un de l'autre, un atome d'hydrogène ou un groupe C₁₋₆-alkyle ;
à l'état de base ou de sel d'addition à un acide.

2. Composé de formule générale (I) selon la revendication 1, **caractérisé en ce que** :
- R₂ représente un phényle, éventuellement substitué par un ou plusieurs atomes d'halogène ou groupes C₁₋₆-alkyle, C₁₋₆-fluoroalkyle.

3. Composé de formule générale (I) selon la revendication 1, **caractérisé en ce que** :
- R₂ représente un groupe choisi parmi les groupes C₁₋₆-alkyle, C₁₋₆-fluoroalkyle, C₃₋₇-cycloalkyle, C₃₋₇-cycloalkyl-C₁₋₆-alkyle.

4. Composé de formule générale (I) selon l'une quelconque des revendications 1 à 3, **caractérisé en ce que** :
- R₇ et R₈ représentent indépendamment l'un de l'autre, un atome d'hydrogène ou un groupe méthyle.

5. Composé de formule générale (I) selon l'une quelconque des revendications 1 à 4, **caractérisé en ce que** :
- A représente un groupe C₁₋₇-alkylène éventuellement substitué par un ou deux groupes Rₐ ;
- B représente un groupe C₁₋₇-alkylène éventuellement substitué par un groupe R_{b} ;
- L représente un atome d'azote éventuellement substitué par un groupe R_{c} ou R_{d},
les atomes de carbone de A et de B étant éventuellement substitués par un ou plusieurs groupes R_{f} identiques ou différents l'un de l'autre ;
- deux groupes Rₐ peuvent former ensemble un groupe C₁₋₆-alkylène ;
- Rₐ et R_{b} peuvent former ensemble une liaison ou un groupe C₁₋₆-alkylène ;
- Rₐ et R_{c} peuvent former ensemble une liaison ou un groupe C₁₋₆-alkylène ;
- R_{b} et R_{c} peuvent former ensemble une liaison ou un groupe C₁₋₆-alkylène ;
- R_{d} représente un groupe choisi parmi l'atome d'hydrogène et les groupes C₁₋₆-alkyle, C₃₋₇-cycloalkyle, C₃₋₇-cycloalkyl-C₁₋₆-alkyle, hydroxy-C₁₋₆-alkyle, C₁₋₆-alkyloxy-C₁₋₆-alkyle, C₁₋₆-alkylthio-C-₁₋₆-alkyle, C₁₋₆-fluoroalkyle, benzyle ;
- R_{f} représente un groupe C₁₋₆-alkyle, C₃₋₇-cycloalkyle, C₃₋₇-cycloalkyle-C₁₋₆-alkyle, C₁₋₆-alkyloxy-C₁₋₆-alkyle, hydroxy-C₁₋₆-alkyle, C₁₋₆-fluoroalkyle ou phényle.

6. Composé de formule générale (I) selon l'une quelconque des revendications 1 à 4, **caractérisé en ce que** :
- A représente un groupe C₁₋₇-alkylène éventuellement substitué par un ou deux groupes Rₐ ;
- B représente un groupe C₁₋₇-alkylène éventuellement substitué par un groupe R_{b} ;
- L représente un atome de carbone substitué par deux groupes Rₑ₂ ;
les atomes de carbone de A et de B étant éventuellement substitués par un ou plusieurs groupes R_{f} identiques ou différents l'un de l'autre ;
- Deux Rₑ₂ forment avec l'atome de carbone qui les porte une monoamine cyclique comportant éventuellement un atome d'oxygène, cette monoamine cyclique étant éventuellement substituée par un ou plusieurs groupes R_{f} identiques ou différents l'un de l'autre ;
- R_{f} représente un groupe C₁₋₆-alkyle.

7. Composé de formule générale (I) selon l'une quelconque des revendications 1 à 4, **caractérisé en ce que** :
- A représente un groupe C₁₋₇-alkylène;
- B représente un groupe C₁₋₇-alkylène;
- L représente un atome de carbone substitué par un groupe Rₑ₁ et un groupe R_{d} ;
- R_{d} représente un atome d'hydrogène ;
- Rₑ₁ représente un groupe -NR₄R₅ ou une monoamine cyclique comportant éventuellement un atome d'oxygène, la monoamine cyclique étant éventuellement substituée par un ou plusieurs R_{f} identiques ou différents l'un de l'autre ;
- R_{f} représente un groupe C₁₋₆-alkyle, C₃₋₇-cycloalkyle, C₃₋₇-cycloalkyle-C₁₋₆-alkyle.

8. Composé de formule générale (I) selon l'une quelconque des revendications 1, 3, 4 et 5, **caractérisé en ce que** :
- R₂ représente un groupe méthyle ;
- l'amine cyclique formée par -N-A-L-B- représente un groupe (R)-3-méthylpipérazin-1-yle, 3,3-diméthyl-pipérazin-1-yle, (*cis*)-3,5-diméthyl-pipérazin-1-yle, 4-isopropyl-pipérazin-1-yle, (*cis*)-5-méthyl-hexahydro-pyrrolo[3,4-c]pyrrol-2(1*H*)-yle ;
- R₇ et R₈ représentent un atome d'hydrogène.

9. Composé de formule générale (I) selon l'une quelconque des revendications 1, 2, 4 et 5, **caractérisé en ce que** :
- R₂ représente un 3-fluoro-phényle ou un 4-fluoro-phényle ;
- l'amine cyclique formée par -N-A-L-B- représente un groupe (*R*)-3-méthylpipérazin-1-yle, 3,3-diméthylpipérazin-1-yle, (*cis*)-3,5-diméthylpipérazin-1-yle, 4-isopropyl-pipérazin-1-yle, 6,9-diaza-spiro[4.5]déc-9-yle, 3-phényl-pipérazin-1-yle, 4-benzyl-pipérazin-1-yle, 3-hydroxyméthyl-pipérazin-1-yle, 4-(2-hydroxy-éthyl)-pipérazin-1-yle, (*R*)-4-(2-hydroxy-propyl)-pipérazin-1-yle, (*S*)-4-(2-hydroxy-propyl)-pipérazin-1-yle, 4-(1-hydroxy-2-méthyl-propan-2-yl)-pipérazin-1-yle, 4-(2-hydroxy-2-méthyl-propyl)-pipérazin-1-yle, 4-(3-hydroxy-3-méthyl-butyl)-pipérazin-1-yle, (*R*)-3-phényl-pipérazin-1-yl, (*S*)-3-phényl-pipérazin-1-yle, 4-benzyl-pipérazin-1-yle, (*cis*)-5-méthyl-hexahydropyrrolo[3,4-c]pyrrol-2(1*H*)-yle, (*cis*)-5-(2-hydroxy-éthyl)-hexahydro-pyrrolo[3,4-c]pyrrol-2(1*H*)-yle, (*4aR*, *7aR*)-1-méthyl-octahydro-6*H-*pyrrolo[3,4-*b*]pyridin-6-yle, (*4aS*, *7aS*)-1-méthyl-octahydro-6*H*-pyrrolo[3,4-*b*]pyridin-6-yle, (1*S*,4*S*)-5-méthyl-2,5-diazabicyclo[2.2.1]hept-2-yle ;
- R₇ et R₈ représentent, indépendamment l'un de l'autre, un atome d'hydrogène ou un groupe méthyle.

10. Composé de formule générale (I) selon l'une quelconque des revendications 1, 2, 4 et 6, **caractérisé en ce que** :
- R₂ représente un groupe 4-fluoro-phényle ;
- l'amine cyclique formée par -N-A-L-B- représente un groupe 2,9-diazaspiro[5.5]undéc-9-yle ;
- R₇ et R₈ représentent un atome d'hydrogène.

11. Composé de formule générale (I) selon l'une quelconque des revendications 1, 2, 4 et 7 **caractérisé en ce que** :
- R₂ représente un groupe 4-fluoro-phényle ;
- l'amine cyclique formée par -N-A-L-B- représente une 4-(pyrrolidin-1-yl)-pipéridin-1-yle ;
- R₇ et R₈ représentent un atome d'hydrogène.

12. Composé selon la revendication 1, choisi parmi :
1. 2-Méthyl-6-[(*R*)-3-méthyl-pipérazin-1-yl]-3-(1*H*-pyrrolo[2,3-b]pyridin-4-yl)-imidazo[1,2-*b*]pyridazine ;
2. 6-(3,3-Diméthyl-pipérazin-1-yl)-2-méthyl-3-(1*H*-pyrrolo[2,3-*b*]pyridin-4-yl)-imidazo[1,2-*b*]pyridazine et son trichlorhydrate ;
3. 6-[(*cis*)-3,5-Diméthyl-pipérazin-1-yl]-2-méthyl-3-(1*H*-pyrrolo[2,3-*b*]pyridin-4-yl)-imidazo[1,2-*b*]pyridazine et son trichlorhydrate ;
4. 6-(4-Isopropyl-pipérazin-1-yl)-2-méthyl-3-(1*H*-pyrrolo[2,3-*b*]pyridin-4-yl)-imidazo[1,2-b]pyridazine et son trichlorhydrate ;
5. 2-Méthyl-6-[(*cis*)-5-méthyl-hexahydro-pyrrolo[3,4-c]pyrrol-2(1*H*)-yl]-3-(1*H-*pyrrolo[2,3-*b*]pyridin-4-yl)-imidazo[1,2-*b*]pyridazine et son trichlorhydrate ;
6. 2-(4-Fluoro-phényl)-6-[(3*R*)-3-méthyl-pipérazin-1-yl]-3-(1*H*-pyrrolo[2,3-*b*]pyridin-4-yl)-imidazo[1,2-*b*]pyridazine ;
7. {4-[2-(4-Fluorophényl)-3-(1*H*-pyrrolo[2,3-*b*]pyridin-4-yl)-imidazo[1,2-*b*]pyridazin-6-yl]-pipérazin-2-yl}-méthanol ;
8. 6-(3,3-Diméthyl-pipérazin-1-yl)-2-(4-fluoro-phényl)-3-(1*H*-pyrrolo[2,3-*b*]pyridin-4-yl)-imidazo[1,2-*b*]pyridazine ;
9. 6-(3,3-Diméthyl-pipérazin-1-yl)-2-(3-fluorophényl)-8-méthyl-3-(1*H*-pyrrolo[2,3-*b*]pyridin-4-yl)-imidazo[1,2-*b*]pyridazine ;
10. 6-(3,3-Diméthyl-pipérazin-1-yl)-2-(4-fluorophényl)-8-méthyl-3-(1*H*-pyrrolo[2,3-*b*]pyridin-4-yl)-imidazo[1,2-*b*]pyridazine ;
11.6-[(*cis*)-3,5-Diméthyl-pipérazin-1-yl]-2-(4-fluoro-phényl)-3-(1*H*-pyrrolo[2,3-*b*]pyridin-4-yl)-imidazo[1,2-*b*]pyridazine ;
12. 2-{4-[2-(3-Fluoro-phényl)-8-méthyl-3-(1*H*-pyrrolo[2,3-*b*]pyridin-4-yl)-imidazo[1,2-*b*]pyridazin-6-yl]-pipérazin-1-yl}-éthanol ;
13. 2-{4-[2-(4-Fluoro-phényl)-8-méthyl-3-(1*H*-pyrrolo[2,3-*b*]pyridin-4-yl)-imidazo[1,2-*b*]pyridazin-6-yl]-pipérazin-1-yl}-éthanol ;
14. 2-(4-Fluoro-phényl)-6-(4-isopropyl-pipérazin-1-yl)-3-(1*H*-pyrrolo[2,3-*b*]pyridin-4-yl)-imidazo[1,2-*b*]pyridazine ;
15. 2-(4-Fluoro-phényl)-6-(4-isopropyl-pipérazin-1-yl)-8-méthyl-3-(1*H*-pyrrolo[2,3-*b*]pyridin-4-yl)-imidazo[1,2-*b*]pyridazine ;
16. (*R*)-1-{4-[2-(4-Fluoro-phényl)-8-méthyl-3-(1*H*-pyrrolo[2,3-*b*]pyridin-4-yl)-imidazo[1,2-*b*]pyridazin-6-yl]-pipérazin-1-yl}-propan-2-ol
17. (*S*)-1-{4-[2-(4-Fluoro-phényl)-8-méthyl-3-(1*H*-pyrrolo[2,3-*b*]pyridin-4-yl)-imidazo[1,2-*b*]pyridazin-6-yl]-pipérazin-1-yl}-propan-2-ol
18. 6-(6,9-Diaza-spiro[4.5]déc-9-yl)-2-(4-fluoro-phényl)-8-méthyl-3-(1*H*-pyrrolo[2,3-*b*]pyridin-4-yl)-imidazo[1,2-*b*]pyridazine
19. 2-{4-[2-(4-Fluoro-phényl)-3-(1*H*-pyrrolo[2,3-*b*]pyridin-4-yl)-imidazo[1,2-*b*]pyridazin-6-yl]-pipérazin-1-yl}-2-méthyl-propan-1-ol
20. 1-{4-[2-(4-Fluoro-phényl)-3-(1*H*-pyrrolo[2,3-*b*]pyridin-4-yl)-imidazo[1,2-*b*]pyridazin-6-yl]-pipérazin-1-yl}-2-méthyl-propan-2-ol ;
21. 1-{4-[2-(3-Fluoro-phényl)-8-méthyl-3-(1*H*-pyrrolo[2,3-*b*]pyridin-4-yl)-imidazo[1,2-*b*]pyridazin-6-yl]-pipérazin-1-yl}-2-méthyl-propan-2-ol ;
22. 1-{4-[2-(4-Fluoro-phényl)-8-méthyl-3-(1*H*-pyrrolo[2,3-*b*]pyridin-4-yl)-imidazo[1,2-*b*]pyridazin-6-yl]-pipérazin-1-yl}-2-méthyl-propan-2-ol ;
23. 4-{4-[2-(4-Fluoro-phényl)-3-(1*H*-pyrrolo[2,3-*b*]pyridin-4-yl)-imidazo[1,2-*b*]pyridazin-6-yl]-pipérazin-1-yl}-2-méthyl-butan-2-ol ;
24. (*R*)-2-(4-Fluoro-phényl)-6-[3-phényl-pipérazin-1-yl]-3-(1*H*-pyrrolo[2,3-*b*]pyridin-4-yl)-imidazo[1,2-*b*]pyridazine et son trichlorhydrate ;
25. (*S*)-2-(4-Fluoro-phényl)-6-[3-phényl-pipérazin-1-yl]-3-(1*H*-pyrrolo[2,3-*b*]pyridin-4-yl)-imidazo[1,2-*b*]pyridazine et son trichlorhydrate ;
26. 2-(4-Fluoro-phényl)-8-méthyl-6-[3-phényl-pipérazin-1-yl]-3-(1*H*-pyrrolo[2,3-*b*]pyridin-4-yl)-imidazo[1,2-*b*]pyridazine ;
27. 6-(4-Benzyl-pipérazin-1-yl)-2-(4-fluoro-phényl)-3-(1*H*-pyrrolo[2,3-*b*]pyridin-4-yl)-imidazo[1,2-*b*]pyridazine ;
28. (*cis*)-2-(4-Fluoro-phényl)-6-(5-méthyl-hexahydro-pyrrolo[3,4-c]pyrrol-2(1*H*)-yl)-3-(1*H-*pyrrolo[2,3-*b*]pyridin-4-yl)-imidazo[1,2-*b*]pyridazine ;
29. (*cis*)-2-(4-Fluoro-phényl)-8-méthyl-6-(5-méthyl-hexahydro-pyrrolo[3,4-c]pyrrol-2(1*H*)-yl)-3-(1*H*-pyrrolo[2,3-*b*]pyridin-4-yl)-imidazo[1,2-*b*]pyridazine ;
30. (*cis*)-2-{5-[2-(4-Fluoro-phényl)-3-(1*H*-pyrrolo[2,3-b]pyridin-4-yl)-imidazo[1,2-*b*]pyridazin-6-yl]-hexahydro-pyrrolo[3,4-c]pyrrol-2(1*H*)-yl}-éthanol ;
31. (*cis*)-2-{5-[2-(4-Fluoro-phényl)-8-méthyl-3-(1*H*-pyrrolo[2,3-*b*]pyridin-4-yl)-imidazo[1,2-*b*]pyridazin-6-yl]-hexahydro-pyrrolo[3,4-c]pyrrol-2(1*H*)-yl}-éthanol ;
32. 2-(4-Fluoro-phényl)-8-méthyl-6-((*4aR*,*7aR*)-1-méthyl-octahydro-6*H*-pyrrolo[3,4-*b*]pyridin-6-yl)-3-(1*H*-pyrrolo[2,3-*b*]pyridin-4-yl)-imidazo[1,2-*b*]pyridazine
33. 2-(4-Fluoro-phényl)-8-méthyl-6-((*4aS*, *7aS*)-1-méthyl-octahydro-6*H-*pyrrolo[3,4-*b*]pyridin-6-yl)-3-(1*H*-pyrrolo[2,3-*b*]pyridin-4-yl)-imidazo[1,2-*b*]pyridazine
34. 2-(4-Fluoro-phényl)-6-((1S,4S)-5-méthyl-2,5-diaza-bicyclo[2.2.1]hept-2-yl)-3-(1*H-*pyrrolo[2,3-*b*]pyridin-4-yl)-imidazo[1,2-*b*]pyridazine ;
35. 9-[2-(4-Fluoro-phényl)-3-(1*H*-pyrrolo[2,3-*b*]pyridin-4-yl)-imidazo[1,2-*b*]pyridazin-6-yl]-2,9-diaza-spiro[5.5]undécane ;
36. 2-(4-Fluoro-phényl)-6-(4-pyrrolidin-1-yl-pipéridin-1-yl)-3-(1*H*-pyrrolo[2,3-*b*]pyridin-4-yl)-imidazo[1,2-*b*]pyridazine.

13. Procédé de préparation d'un composé de formule générale (I) selon la revendication 1, **caractérisé en ce que** l'on fait réagir un composé de formule générale (II) 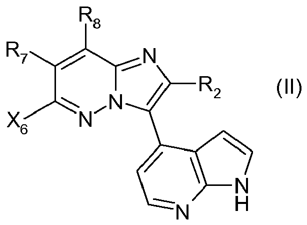 dans laquelle R₂, R₇ et R₈ sont tels que définis selon la revendication 1 et X₆ représente un groupe partant, avec une amine de formule générale (III) 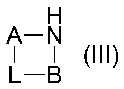 dans laquelle A, L et B sont tel que définis selon la revendication 1.

14. Procédé de préparation d'un composé de formule générale (I) selon la revendication 1, **caractérisé en ce que** l'on déprotège un composé de formule générale (V) 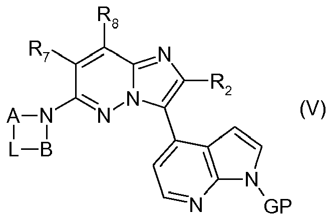 dans laquelle R₂, A, L, B, R₇ et R₈ sont tels que définis selon la revendication 1 et GP représente un groupement benzène ou toluène sulfonyle, au moyen d'une base.

15. Médicament, **caractérisé en ce qu'**il comprend un composé de formule (I) selon l'une quelconque des revendications 1 à 12, à l'état de base ou de sel d'addition à un acide pharmaceutiquement acceptable.

16. Composition pharmaceutique, **caractérisée en ce qu'**elle comprend un composé de formule (I) selon l'une quelconque des revendications 1 à 12, à l'état de base ou de sel d'addition à un acide pharmaceutiquement acceptable, ainsi qu'au moins un excipient pharmaceutiquement acceptable.

17. Utilisation d'un composé de formule générale (I) selon l'une quelconque des revendications 1 à 12, pour la préparation d'un médicament destiné à la prévention ou au traitement des désordres du sommeil, des troubles du rythme circadien, des troubles de l'humeur, des troubles anxieux et dépressifs, des maladies liées à la dépendance à des substances d'abus, des maladies reliées à l'hyperphosphorylation de la protéine tau, des maladies causées ou exacerbées par la prolifération des cellules ou des maladies inflammatoires.

## Patentansprüche

1. Verbindung der allgemeinen Formel (I) 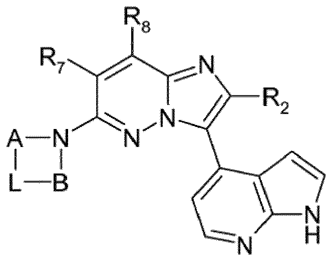 worin
- R₂ für eine Arylgruppe, die gegebenenfalls durch einen oder mehrere Substituenten, die aus Halogenatomen und C₁₋₆-Alkyl-, C₁₋₆-Alkyloxy-, C₁₋₆-Alkylthio, C₁₋₆-Fluoralkyl-, C₁₋₆-Fluoralkyloxy- und
- CN-Gruppen ausgewählt sind, substituiert ist, steht oder R₂ für eine C₁₋₆-Alkyl-, C₁₋₆-Fluoralkyl-, C₃₋₇-Cycloalkyl- oder C₃₋₇-Cycloalkyl-C₁₋₆-alkylgruppe steht;
- A für eine C₁₋₇-Alkylengruppe, die gegebenenfalls durch eine oder zwei Gruppen Rₐ substituiert ist, steht;
- B für eine C₁₋₇-Alkylengruppe, die gegebenenfalls durch eine Gruppe R_{b} substituiert ist, steht;
- L entweder für ein Stickstoffatom, das gegebenenfalls durch eine Gruppe R_{c} oder R_{d} substituiert ist, oder für ein Kohlenstoffatom, das durch eine Gruppe Rₑ₁ und eine Gruppe R_{d} oder zwei Gruppen Rₑ₂ substituiert ist, steht;
wobei die Kohlenstoffatome von A und B gegebenenfalls durch eine oder mehrere Gruppen R_{f}, die gleich oder voneinander verschieden sein können, substituiert sind;
- Rₐ, R_{b} und R_{c} so definiert sind, dass:
zwei Gruppen Rₐ zusammen eine C₁₋₆-Alkylengruppe bilden können;
Rₐ und R_{b}, zusammen eine Bindung oder eine C₁₋₆-Alkylengruppe bilden können;
Rₐ und R_{c} zusammen eine Bindung oder eine C₁₋₆-Alkylengruppe bilden können;
R_{b}, und R_{c} zusammen eine Bindung oder eine C₁₋₆-Alkylengruppe bilden können;
- R_{d} für eine Gruppe steht, die aus einem Wasserstoffatom und C₁₋₆-Alkyl-, C₃₋₇-Cycloalkyl-, C₃₋₇-Cycloalkyl-C₁₋₆-alkyl-, Hydroxy-C₁₋₆-alkyl-, C₁₋₆-Alkyloxy-C₁₋₆-alkyl-, C₁₋₆-Alkylthio-C₁₋₆-alkyl-, C₁₋₆-Fluoralkyl- und Benzylgruppen ausgewählt ist;
- Rₑ₁ für eine Gruppe -NR₄R₅ oder ein cyclisches Monoamin, das gegebenenfalls ein Sauerstoffatom umfasst, steht, wobei das cyclische Monoamin gegebenenfalls durch einen oder mehrere Substituenten, die aus einem Fluoratom und C₁₋₆-Alkyl-, C₁₋₆-Alkyloxy- und Hydroxylgruppen ausgewählt sind, substituiert ist;
- zwei Gruppen Rₑ₂ mit dem Kohlenstoffatom, das sie trägt, ein cyclisches Monoamin, das gegebenenfalls ein Sauerstoffatom umfasst, bilden, wobei dieses cyclische Monoamin gegebenenfalls durch eine oder mehrere Gruppen R_{f}, die gleich oder voneinander verschieden sein können, substituiert ist;
- R_{f} für eine C₁₋₆-Alkyl-, C₃₋₇-Cycloalkyl-, C₃₋₇-Cycloalkyl-C₁₋₆-alkyl-, C₁₋₆-Alkyloxy-C₁₋₆-alkyl-, Hydroxy-C₁₋₆-alkyl-, C₁₋₆-Fluoralkyl-, Phenyl- oder Benzylgruppe steht;
- R₄ und R₅ unabhängig voneinander für ein Wasserstoffatom oder eine C₁₋₆-Alkyl-, C₃₋₇-Cycloalkyl- oder C₃₋₇-Cycloalkyl-C₁₋₆-alkylgruppe stehen;
- R₇ und R₈ unabhängig voneinander für ein Wasserstoffatom oder eine C₁₋₆-Alkylgruppe stehen; in Basen- oder Säureadditionssalzform.

2. Verbindung der allgemeinen Formel (I) nach Anspruch 1, **dadurch gekennzeichnet, dass**:
- R₂ für ein Phenyl, das gegebenenfalls durch ein oder mehrere Halogenatome oder eine oder mehrere C₁₋₆-Alkyl- oder C₁₋₆-Fluoralkylgruppen substituiert ist.

3. Verbindung der allgemeinen Formel (I) nach Anspruch 1, **dadurch gekennzeichnet, dass**:
- R₂ für eine Gruppe, die aus C₁₋₆-Alkyl-, C₁₋₆-Fluoralkyl-, C₃₋₇-Cycloalkyl- oder C₃₋₇-Cycloalkyl-C₁₋₆-alkylgruppen ausgewählt ist, steht.

4. Verbindung der allgemeinen Formel (I) nach einem der Ansprüche 1 bis 3, **dadurch gekennzeichnet, dass**:
- R₇ und R₈ unabhängig voneinander für ein Wasserstoffatom oder eine Methylgruppe stehen.

5. Verbindung der allgemeinen Formel (I) nach einem der Ansprüche 1 bis 4, **dadurch gekennzeichnet, dass**:
- A für eine C₁₋₇-Alkylengruppe, die gegebenenfalls durch eine oder zwei Gruppen Rₐ substituiert ist, steht;
- B für eine C₁₋₇-Alkylengruppe, die gegebenenfalls durch eine Gruppe R_{b} substituiert ist, steht;
- L für ein Stickstoffatom, das gegebenenfalls durch eine Gruppe R_{c} oder R_{d} substituiert ist, steht;
wobei die Kohlenstoffatome von A und B gegebenenfalls durch eine oder mehrere Gruppen R_{f}, die gleich oder voneinander verschieden sein können, substituiert sind;
- zwei Gruppen Rₐ zusammen eine C₁₋₆-Alkylengruppe bilden können;
- Rₐ und R_{b} zusammen eine Bindung oder eine C₁₋₆-Alkylengruppe bilden können;
- Rₐ und R_{c} zusammen eine Bindung oder eine C₁₋₆-Alkylengruppe bilden können;
- R_{b} und R_{c} zusammen eine Bindung oder eine C₁₋₆-Alkylengruppe bilden können;
- R_{d} für eine Gruppe steht, die aus einem Wasserstoffatom und C₁₋₆-Alkyl-, C₃₋₇-Cycloalkyl-, C₃₋₇-Cycloalkyl-C₁₋₆-alkyl-, Hydroxy-C₁₋₆-alkyl-, C₁₋₆-Alkyloxy-C₁₋₆-alkyl-, C₁₋₆-Alkylthio-C₁₋₆-alkyl-, C₁₋₆-Fluoralkyl- und Benzylgruppen ausgewählt ist;
- R_{f} für eine C₁₋₆-Alkyl-, C₃₋₇-Cycloalkyl-, C₃₋₇-Cycloalkyl-C₁₋₆-alkyl-, C₁₋₆-Alkyloxy-C₁₋₆-alkyl-, Hydroxy-C₁₋₆-alkyl-, C₁₋₆-Fluoralkyl- oder Phenylgruppe steht.

6. Verbindung der allgemeinen Formel (I) nach einem der Ansprüche 1 bis 4, **dadurch gekennzeichnet, dass**:
- A für eine C₁₋₇-Alkylengruppe, die gegebenenfalls durch eine oder zwei Gruppen Rₐ substituiert ist, steht;
- B für eine C₁₋₇-Alkylengruppe, die gegebenenfalls durch eine Gruppe R_{b} substituiert ist, steht;
- L für ein Kohlenstoffatom, das durch zwei Gruppen Rₑ₂ substituiert ist, steht;
wobei die Kohlenstoffatome von A und B gegebenenfalls durch eine oder mehrere Gruppen R_{f}, die gleich oder voneinander verschieden sein können, substituiert sind;
- zwei Gruppen Rₑ₂ mit dem Kohlenstoffatom, das sie trägt, ein cyclisches Monoamin, das gegebenenfalls ein Sauerstoffatom umfasst, bilden, wobei dieses cyclische Monoamin gegebenenfalls durch eine oder mehrere Gruppen R_{f}, die gleich oder voneinander verschieden sein können, substituiert ist;
- R_{f} für eine C₁₋₆-Alkylgruppe steht.

7. Verbindung der allgemeinen Formel (I) nach einem der Ansprüche 1 bis 4, **dadurch gekennzeichnet, dass**:
- A für eine C₁₋₇-Alkylengruppe steht;
- B für eine C₁₋₇-Alkylengruppe steht;
- L für ein Kohlenstoffatom, das durch eine Gruppe Rₑ₁ und eine Gruppe R_{d} substituiert ist, steht;
- R_{d} für ein Wasserstoffatom steht;
- Rₑ₁ für eine Gruppe -NR₄R₅ oder ein cyclisches Monoamin, das gegebenenfalls ein Sauerstoffatom umfasst, steht, wobei das cyclische Monoamin gegebenenfalls durch eine oder mehrere Gruppen R_{f}, die gleich oder voneinander verschieden sind, substituiert ist;
- R_{f} für eine C₁₋₆-Alkyl-, C₃₋₇-Cycloalkyl- oder C₃₋₇-Cycloalkyl-C₁₋₆-alkylgruppe steht.

8. Verbindung der allgemeinen Formel (I) nach einem der Ansprüche 1, 3, 4 und 5, **dadurch gekennzeichnet, dass**:
- R₂ für eine Methylgruppe steht;
- das durch -N-A-L-B- gebildete cyclische Amin für eine (*R*)-3-Methylpiperazin-1-yl-, 3,3-Dimethylpiperazin-1-yl-, (*cis*)-3,5-Dimethyl-piperazin-1-yl, 4-Isopropylpiperazin-1-yl- oder (*cis*)-5-Methylhexahydropyrrolo[3,4-c]pyrrol-2-(1*H*)-ylgruppe steht;
- R₇ und R₈ für ein Wasserstoffatom stehen.

9. Verbindung der allgemeinen Formel (I) nach einem der Ansprüche 1, 2, 4 und 5, **dadurch gekennzeichnet, dass**:
- R₂ für eine 3-Fluorphenyl- oder 4-Fluorphenylgruppe steht;
- das durch -N-A-L-B- gebildete cyclische Amin für eine (*R*)-3-Methylpiperazin-1-yl-, 3,3-Dimethylpiperazin-1-yl-, (*cis*)-3,5-Dimethyl-piperazin-1-yl, 4-Isopropylpiperazin-1-yl-, 6,9-Diazaspiro[4.5]dec-9-yl-, 3-Phenylpiperazin-1-yl-, 4-Benzylpiperazin-1-yl-, 3-Hydroxymethylpiperazin-1-yl-, 4-(2-Hydroxyethyl)piperazin-1-yl-, (R)-4-(2-Hydroxypropyl)piperazin-1-yl-, (*S*)-4-(2-Hydroxypropyl)piperazin-1-yl-, 4-(1-Hydroxy-2-methylpropan-2-yl)piperazin-1-yl-, 4-(2-Hydroxy-2-methylpropyl)piperazin-1-yl-, 4-(3-Hydroxy-3-methylbutyl)piperazin-1-yl-, *(R)*-3-Phenylpiperazin-1-yl-, (*S*)-3-Phenylpiperazin-1-yl-, 4-Benzylpiperazin-1-yl-, (*cis*)-5-Methylhexahydropyrrolo[3,4-*c*]pyrrol-2(1*H*)-yl-, (cis)-5-(2-Hydroxyethyl)hexahydropyrrolo[3,4-c]pyrrol-2(1*H*)-yl-, (*4aR,7aR*)-1-Methyloctahydro-6*H*-pyrrolo[3,4-*b*]pyridin-6-yl-, (*4aS,7aS*)-1-Methyloctahydro-6*H*-pyrrolo[3,4-*b*]pyridin-6-yl- oder (1*S*,4*S*)-5-Methyl-2,5-diazabicyclo[2.2.1]hept-1-ylgruppe steht;
- R₇ und R₈ jeweils unabhängig voneinander für ein Wasserstoffatom oder eine Methylgruppe stehen.

10. Verbindung der allgemeinen Formel (I) nach einem der Ansprüche 1, 2, 4 und 6, **dadurch gekennzeichnet, dass**:
- R₂ für eine 4-Fluorphenylgruppe steht;
- das durch -N-A-L-B- gebildete cyclische Amin für eine 2,9-Diazaspiro[5.5]undec-9-ylgruppe steht;
- R₇ und R₈ für ein Wasserstoffatom stehen.

11. Verbindung der allgemeinen Formel (I) nach einem der Ansprüche 1, 2, 4 und 7, **dadurch gekennzeichnet, dass**:
- R₂ für eine 4-Fluorphenylgruppe steht;
- das durch -N-A-L-B- gebildete cyclische Amin für ein 4-(Pyrrolidin-1-yl)piperidin-1-yl steht;
- R₇ und R₈ für ein Wasserstoffatom stehen.

12. Verbindung nach Anspruch 1, ausgewählt aus:
1. 2-Methyl-6-[(*R*)-3-methylpiperazin-1-yl]-3-(1*H-*pyrrolo[2,3-*b*]pyridin-4-yl)imidazo[1,2-*b*]pyridazin;
2. 6-(3,3-Dimethylpiperazin-1-yl)-2-methyl-3-(1H-pyrrolo[2,3-*b*]pyridin-4-yl)imidazo[1,2-*b*]pyridazin und dem Trihydrochlorid davon;
3. 6-[(*cis*)-3,5-Dimethylpiperazin-1-yl]-2-methyl-3-(1*H*-pyrrolo[2,3-*b*]pyridin-4-yl)imidazo[1,2-*b*]pyridazin und dem Trihydrochlorid davon;
4. 6-(4-Isopropylpiperazin-1-yl)-2-methyl-3-(1*H-*pyrrolo[2,3-*b*]pyridin-4-yl)imidazo[1,2-*b*]pyridazin und dem Trihydrochlorid davon;
5. 2-Methyl-6-[(cis)-5-methylhexahydropyrrolo[3,4-*c*]pyrrol-2(1*H*)-yl]-3-(1*H*-pyrrolo[2,3-*b*]pyridin-4-yl)imidazo[1,2-*b*]pyridazin und dem Trihydrochlorid davon;
6. 2-(4-Fluorphenyl)-6-[(3R)-3-methylpiperazin-1-yl]-3-(1*H*-pyrrolo[2,3-*b*]pyridin-4-yl)-imidazo[1,2-b]pyridazin;
7. {4-[2-(4-Fluorphenyl)-3-(1*H*-pyrrolo[2,3-*b*]pyridin-4-yl)imidazo[1,2-b]pyridazin-6-yl]-piperazin-2-yl}methanol;
8. 6-(3,3-Dimethylpiperazin-1-yl)-2-(4-fluorphenyl)-3-(1*H*-pyrrolo[2,3-*b*]pyridin-4-yl)imidazo[1,2-b]pyridazin;
9. 6-(3,3-Dimethylpiperazin-1-yl)-2-(3-fluorphenyl)-8-methyl-3-(1*H*-pyrrolo[2,3-*b*]pyridin-4-yl)imidazo[1,2-*b*]pyridazin;
10. 6-(3,3-Dimethylpiperazin-1-yl)-2-(4-fluorphenyl)-8-methyl-3-(1*H*-pyrrolo[2,3-*b*]pyridin-4-yl)imidazo[1,2-*b*]pyridazin;
11. 6-[(*cis*)-3,5-Dimethylpiperazin-1-yl]-2-(4-fluorphenyl)-3-(1*H*-pyrrolo[2,3-*b*]pyridin-4-yl)imidazo[1,2-b]pyridazin;
12. 2-{4-[2-(3-Fluorphenyl)-8-methyl-3-(1*H-*pyrrolo[2,3-*b*]pyridin-4-yl)imidazo[1,2-*b*]pyridazin-6-yl]piperazin-1-yl}ethanol;
13. 2-{4-[2-(4-Fluorphenyl)-8-methyl-3-(1*H-*pyrrolo[2,3-*b*]pyridin-4-yl)imidazo[1,2-*b*]pyridazin-6-yl]piperazin-1-yl}ethanol;
14. 2-(4-Fluorphenyl)-6-(4-isopropylpiperazin-1-yl)-3-(1*H*-pyrrolo[2,3-*b*]pyridin-4-yl)-imidazo[1,2-*b*]pyridazin;
15. 2-(4-Fluorphenyl)-6-(4-isopropylpiperazin-1-yl)-8-methyl-3-(1*H*-pyrrolo[2,3-*b*]pyridin-4-yl)imidazo[1,2-b]pyridazin;
16. (*R*)-1-{4-[2-(4-Fluorphenyl)-8-methyl-3-(1H-pyrrolo[2,3-*b*]pyridin-4-yl)imidazo[1,2-*b*]pyridazin-6-yl]piperazin-1-yl}propan-2-ol;
17. (*S*)-1-{4-[2-(4-Fluorphenyl)-8-methyl-3-(1H-pyrrolo[2,3-*b*]pyridin-4-yl)imidazo[1,2-*b*]pyridazin-6-yl]piperazin-1-yl}propan-2-ol;
18. 6-(6,9-Diazaspiro[4.5]dec-9-yl)-2-(4-fluorphenyl)-8-methyl-3-(1*H*-pyrrolo[2,3-*b*]pyridin-4-yl)imidazo[1,2-*b*]pyridazin;
19. 2-{4-[2-(4-Fluorphenyl)-3-(1*H*-pyrrolo[2,3-*b*]pyridin-4-yl)imidazo[1,2-*b*]pyridazin-6-yl]piperazin-1-yl}-2-methylpropan-1-ol;
20. 1-{4-[2-(4-Fluorphenyl)-3-(1*H*-pyrrolo[2,3-*b*]pyridin-4-yl)imidazo[1,2-*b*]pyridazin-6-yl]piperazin-1-yl}-2-methylpropan-2-ol;
21. 1-{4-[2-(3-Fluorphenyl)-8-methyl-3-(1*H-*pyrrolo[2,3-*b*]pyridin-4-yl)imidazo[1,2-*b*]pyridazin-6-yl]piperazin-1-yl}-2-methylpropan-2-ol;
22. 1-{4-[2-(4-Fluorphenyl)-8-methyl-3-(1*H-*pyrrolo[2,3-*b*]pyridin-4-yl)imidazo[1,2-*b*]pyridazin-6-yl]piperazin-1-yl}-2-methylpropan-2-ol;
23. 4-{4-[2-(4-Fluorphenyl)-3-(1*H*-pyrrolo[2,3-*b*]pyridin-4-yl)imidazo[1,2-*b*]pyridazin-6-yl]piperazin-1-yl}-2-methylbutan-2-ol;
24. (*R*)-2-(4-Fluorphenyl)-6-[3-phenylpiperazin-1-yl]-3-(1*H*-pyrrolo[2,3-*b*]pyridin-4-yl)-imidazo[1,2-*b*]pyridazin und dem Trihydrochlorid davon;
25. (*S*)-2-(4-Fluorphenyl)-6-[3-phenylpiperazin-1-yl]-3-(1*H*-pyrrolo[2,3-*b*]pyridin-4-yl)-imidazo[1,2-*b*]pyridazin und dem Trihydrochlorid davon;
26. 2-(4-Fluorphenyl)-8-methyl-6-[3-phenylpiperazin-1-yl]-3-(1*H*-pyrrolo[2,3-*b*]pyridin-4-yl)imidazo[1,2-*b*]pyridazin;
27. 6-(4-Benzylpiperazin-1-yl)-2-(4-fluorphenyl)-3-(1*H*-pyrrolo[2,3-*b*]pyridin-4-yl)imidazo[1,2-*b*]pyridazin;
28. (*cis*)-2-(4-Fluorphenyl)-6-(5-methylhexahydropyrrolo[3,4-*c*]pyrrol-2(1*H*)-yl)-3-(1*H*-pyrrolo[2,3-*b*]pyridin-4-yl)imidazo[1,2-*b*]pyridazin;
29. (*cis*)-2-(4-Fluorphenyl)-8-methyl-6-(5-methylhexahydropyrrolo[3,4-*c*]pyrrol-2(1*H*)-yl)-3-(1*H*-pyrrolo[2,3-*b*]pyridin-4-yl)imidazo[1,2-*b*]pyridazin;
30. (*cis*)-2-{5-[2-(4-Fluorphenyl)-3-(1*H-*pyrrolo[2,3-*b*]pyridin-4-yl)imidazo[1,2-*b*]pyridazin-6-yl]hexahydropyrrolo[3,4-*c*]pyrrol-2(1*H*)-yl}ethanol;
31. (*cis*)-2-{5-[2-(4-Fluorphenyl)-8-methyl-3-(1H-pyrrolo[2,3-*b*]pyridin-4-yl)imidazo[1,2-*b*]pyridazin-6-yl]hexahydropyrrolo[3,4-c]pyrrol-2(1*H*)-yl}ethanol;
32. 2-(4-Fluorphenyl)-8-methyl-6-((*4aR*,*7aR*)-1-methyloctahydro-6*H*-pyrrolo[3,4-*b*]pyridin-6-yl)-3-(1*H*-pyrrolo[2,3-*b*]pyridin-4-yl)imidazo[1,2-*b*]pyridazin;
33. 2-(4-Fluorphenyl)-8-methyl-6-((*4aS*,7*aS*)-1-methyloctahydro-6*H*-pyrrolo[3,4-*b*]pyridin-6-yl)-3-(1*H*-pyrrolo[2,3-*b*]pyridin-4-yl)imidazo[1,2-*b*]pyridazin;
34. 2-(4-Fluorphenyl)-6-((1*S*,4*S*)-5-methyl-2,5-diazabicyclo[2.2.1]hept-2-yl)-3-(1H-pyrrolo[2,3-*b*]pyridin-4-yl)imidazo[1,2-*b*]pyridazin;
35. 9-[2-(4-Fluorphenyl)-3-(1*H*-pyrrolo[2,3-*b*]pyridin-4-yl)imidazo[1,2-*b*]pyridazin-6-yl]-2,9-diazaspiro[5.5]undecan;
36. 2-(4-Fluorphenyl)-6-(4-pyrrolidin-1-ylpiperidin-1-yl)-3-(1*H*-pyrrolo[2,3-*b*]pyridin-4-yl)imidazo[1,2-*b*]pyridazin.

13. Verfahren zur Herstellung einer Verbindung der allgemeinen Formel (I) nach Anspruch 1, **dadurch gekennzeichnet, dass** man eine Verbindung der allgemeinen Formel (II) 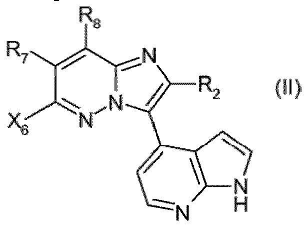 worin R₂, R₇ und R₈ wie in Anspruch 1 definiert sind und X₆ für eine Abgangsgruppe steht, mit einem Amin der allgemeinen Formel (III) 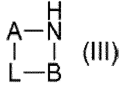 worin A, L und B wie in Anspruch 1 definiert sind, umsetzt.

14. Verfahren zur Herstellung einer Verbindung der allgemeinen Formel (I) nach Anspruch 1, **dadurch gekennzeichnet, dass** man eine Verbindung der allgemeinen Formel (V) 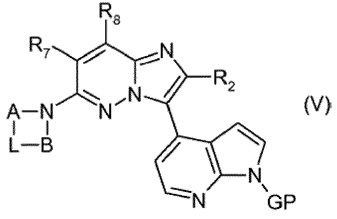 worin R₂, A, L, B, R₇ und R₈ wie in Anspruch 1 definiert sind, und GP für eine Benzol- oder Toluolsulfonylgruppe steht, mit Hilfe einer Base entschützt.

15. Medikament, **dadurch gekennzeichnet, dass** es eine Verbindung der Formel (I) nach einem der Ansprüche 1 bis 12 in Basenform oder in Form eines Additionssalzes mit einer pharmazeutisch unbedenklichen Säure umfasst.

16. Pharmazeutische Zusammensetzung, **dadurch gekennzeichnet, dass** sie eine Verbindung der Formel (I) nach einem der Ansprüche 1 bis 12 in Basenform oder in Form eines Additionssalzes mit einer pharmazeutisch unbedenklichen Säure sowie mindestens einen pharmazeutisch unbedenklichen Hilfsstoff umfasst.

17. Verwendung einer Verbindung der allgemeinen Formel (I) nach einem der Ansprüche 1 bis 12 zur Herstellung eines Medikaments zur Prävention oder Behandlung von Schlafstörungen, Störungen des zirkadianen Rhythmus, Gemütskrankheiten, Angst- und Depressionsstörungen, Erkrankungen, die mit Drogenabhängigkeit assoziiert sind, Erkrankungen, die mit der Hyperphosphorylierung des Tau-Proteins in Verbindung stehen, Erkrankungen, die durch Zellproliferation verursacht oder verschlimmert werden, oder Entzündungserkrankungen.

## Claims

1. Compound of general formula (I): 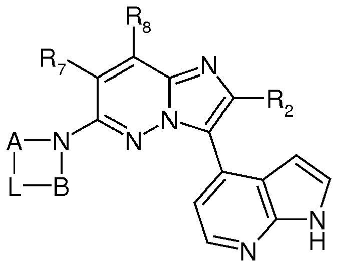 in which:
- R₂ represents an aryl group optionally substituted with one or more substituents chosen from halogen atoms and C₁₋₆-alkyl, C₁₋₆-alkyloxy, C₁₋₆-alkylthio, C₁₋₆-fluoroalkyl, C₁₋₆-fluoroalkyloxy and -CN groups or R₂ represents a C₁₋₆-alkyl, C₁₋₆-fluoroalkyl, C₃₋₇-cycloalkyl or C₃₋₇-cycloalkyl-C₁₋₆-alkyl group;
- A represents a C₁₋₇-alkylene group optionally substituted with one or two Rₐ groups;
- B represents a C₁₋₇-alkylene group optionally substituted with an R_{b} group;
- L represents either a nitrogen atom optionally substituted with an R_{c} or R_{d} group, or a carbon atom substituted with an Rₑ₁ group and an R_{d} group or two Rₑ₂ groups;
the carbon atoms of A and B being optionally substituted with one or more R_{f} groups, which may be identical to or different from one another;
- Rₐ, R_{b} and R_{c} are defined such that:
two Rₐ groups may together form a C₁₋₆-alkylene group;
Rₐ and R_{b} may together form a bond or a C₁₋₆-alkylene group;
Rₐ and R_{c} may together form a bond or a C₁₋₆-alkylene group;
R_{b} and R_{c} may together form a bond or a C₁₋₆-alkylene group;
- R_{d} represents a group chosen from a hydrogen atom and C₁₋₆-alkyl, C₃₋₇-cycloalkyl, C₃₋₇-cycloalkyl-C₁₋₆-alkyl, hydroxy-C₁₋₆-alkyl, C₁₋₆-alkyloxy-C₁₋₆-alkyl, C₁₋₆-alkylthio-C₁₋₆-alkyl, C₁₋₆-fluoroalkyl or benzyl groups;
- Rₑ₁ represents an -NR₄R₅ group or a cyclic monoamine optionally comprising an oxygen atom, the cyclic monoamine being optionally substituted with one or more substituents chosen from a fluorine atom and C₁₋₆-alkyl, C₁₋₆-alkyloxy and hydroxyl groups;
- two Rₑ₂ groups form, with the carbon atom that bears them, a cyclic monoamine optionally comprising an oxygen atom, the cyclic monoamine being optionally substituted with one or more R_{f} groups, which may be identical to or different from one another;
- R_{f} represents a C₁₋₆-alkyl, C₃₋₇-cycloalkyl, C₃₋₇-cycloalkyl-C₁₋₆-alkyl, C₁₋₆-alkyloxy-C₁₋₆-alkyl, hydroxy-C₁₋₆-alkyl, C₁₋₆-fluoroalkyl, phenyl or benzyl group;
- R₄ and R₅ represent, independently of one another, a hydrogen atom or a C₁₋₆-alkyl, C₃₋₇-cycloalkyl or C₃₋₇-cycloalkyl-C₁₋₆-alkyl group; and
- R₇ and R₈ represent, independently of one another, a hydrogen atom or a C₁₋₆-alkyl group;
in the form of a base or of an addition salt with an acid.

2. Compound of general formula (I) according to Claim 1, **characterized in that**:
- R₂ represents a phenyl optionally substituted with one or more halogen atoms or C₁₋₆-alkyl or C₁₋₆-fluoroalkyl groups.

3. Compound of general formula (I) according to Claim 1, **characterized in that**:
- R₂ represents a group chosen from C₁₋₆-alkyl, C₁₋₆-fluoroalkyl, C₃₋₇-cycloalkyl or C₃₋₇-cycloalkyl-C₁₋₆-alkyl groups.

4. Compound of general formula (I) according to any one of Claims 1 to 3, **characterized in that**:
- R₇ and R₈ represent, independently of each other, a hydrogen atom or a methyl group.

5. Compound of general formula (I) according to any one of Claims 1 to 4, **characterized in that**:
- A represents a C₁₋₇-alkylene group optionally substituted with one or two Rₐ groups;
- B represents a C₁₋₇-alkylene group optionally substituted with an R_{b} group;
- L represents a nitrogen atom optionally substituted with an R_{c} or R_{d} group;
the carbon atoms of A and of B being optionally substituted with one or more R_{f} groups, which may be identical to or different from each other;
- two Rₐ groups may together form a C₁₋₆-alkylene group;
- Rₐ and R_{b} may together form a bond or a C₁₋₆-alkylene group;
- Rₐ and R_{c} may together form a bond or a C₁₋₆-alkylene group;
- R_{b} and R_{c} may together form a bond or a C₁₋₆-alkylene group;
- R_{d} represents a group chosen from a hydrogen atom and C₁₋₆-alkyl, C₃₋₇-cycloalkyl, C₃₋₇-cycloalkyl-C₁₋₆-alkyl, hydroxy-C₁₋₆-alkyl, C₁₋₆-alkyloxy-C₁₋₆-alkyl, C₁₋₆-alkylthio-C₁₋₆-alkyl, C₁₋₆-fluoroalkyl or benzyl groups; and
- R_{f} represents a C₁₋₆-alkyl, C₃₋₇-cycloalkyl, C₃₋₇-cycloalkyl-C₁₋₆-alkyl, C₁₋₆-alkyloxy-C₁₋₆-alkyl, hydroxy-C₁₋₆-alkyl, C₁₋₆-fluoroalkyl or phenyl group.

6. Compound of general formula (I) according to any one of Claims 1 to 4, **characterized in that**:
- A represents a C₁₋₇-alkylene group optionally substituted with one or two Rₐ groups;
- B represents a C₁₋₇-alkylene group optionally substituted with an R_{b} group;
- L represents a carbon atom optionally substituted with two Rₑ₂ groups;
the carbon atoms of A and of B being optionally substituted with one or more R_{f} groups, which may be identical to or different from each other;
- two Rₑ₂ groups form, with the carbon atom that bears them, a cyclic monoamine optionally comprising an oxygen atom, this cyclic monoamine being optionally substituted with one or more R_{f} groups, which may be identical to or different from one another; and
- R_{f} represents a C₁₋₆-alkyl group.

7. Compound of general formula (I) according to any one of Claims 1 to 4, **characterized in that**:
- A represents a C₁₋₇-alkylene group;
- B represents a C₁₋₇-alkylene group;
- L represents a carbon atom substituted with an Rₑ₁ group and an R_{d} group;
- R_{d} represents a hydrogen atom;
- Rₑ₁ represents an -NR₄R₅ group or a cyclic monoamine optionally comprising an oxygen atom, the cyclic monoamine being optionally substituted with one or more R_{f} groups, which may be identical to or different from one another; and
- R_{f} represents a C₁₋₆-alkyl, C₃₋₇-cycloalkyl or C₃₋₇-cycloalkyl-C₁₋₆-alkyl group.

8. Compound of general formula (I) according to any one of Claims 1, 3, 4 and 5, **characterized in that**:
- R₂ represents a methyl group;
- the cyclic amine formed by -N-A-L-B- represents an (*R*)-3-methylpiperazin-1-yl, 3,3-dimethylpiperazin-1-yl, (*cis*)-3,5-dimethylpiperazin-1-yl, 4-isopropylpiperazin-1-yl or (*cis*)-5-methylhexahydropyrrolo[3,4-*c*]pyrrol-2(1*H*)-yl group; and
- R₇ and R₈ represent a hydrogen atom.

9. Compound of general formula (I) according to any one of Claims 1, 2, 4 and 5, **characterized in that**:
- R₂ represents a 3-fluorophenyl or a 4-fluorophenyl group;
- the cyclic amine formed by -N-A-L-B- represents an (*R*)-3-methylpiperazin-1-yl, 3,3-dimethylpiperazin-1-yl, (*cis*)-3,5-dimethylpiperazin-1-yl, 4-isopropylpiperazin-1-yl, 6,9-diazaspiro[4.5]dec-9-yl, 3-phenylpiperazin-1-yl, 4-benzylpiperazin-1-yl, 3-hydroxymethylpiperazin-1-yl, 4-(2-hydroxyethyl)piperazin-1-yl, (*R*)-4-(2-hydroxypropyl)piperazin-1-yl, *(S)-*4*-*(2-hydroxypropyl)piperazin-1-yl, 4-(1-hydroxy-2-methylpropan-2-yl)piperazin-1-yl, 4-(2-hydroxy-2-methylpropyl)piperazin-1-yl, 4-(3-hydroxy-3-methylbutyl)piperazin-1-yl, (*R*)-3-phenylpiperazin-1-yl, (*S*)-3-phenylpiperazin-1-yl, 4-benzylpiperazin-1-yl, (*cis*)-5-methylhexahydropyrrolo[3,4-c]pyrrol-2(1*H*)-yl, (*cis*)-5-(2-hydroxyethyl)hexahydropyrrolo[3,4-*c*]pyrrol-2(1*H*)-yl, (*4aR*,*7aR*)-1-methyloctahydro-6*H*-pyrrolo[3,4-*b*]pyridin-6-yl, (*4aS,7aS*)-1-methyloctahydro-6*H-*pyrrolo[3,4-*b*]pyridin-6-yl, or (1*S*,4*S*)-5-methyl-2,5-diazabicyclo[2.2.1]hept-2-yl group; and
- R₇ and R₈ represent, independently of each other, a hydrogen atom or a methyl group.

10. Compound of general formula (I) according to any one of Claims 1, 2, 4 and 6, **characterized in that**:
- R₂ represents a 4-fluorophenyl group;
- the cyclic amine formed by -N-A-L-B- represents a 2,9-diazaspiro[5.5]undec-9-yl group; and
- R₇ and R₈ represent a hydrogen atom.

11. Compound of general formula (I) according to any one of Claims 1, 2, 4 and 7, **characterized in that**:
- R₂ represents a 4-fluorophenyl group;
- the cyclic amine formed by -N-A-L-B- represents a 4-(pyrrolidin-1-yl)-piperidin-1-yl group;
- R₇ and R₈ represent a hydrogen atom..

12. Compound according to Claim 1, chosen from:
1. 2-Methyl-6-[(*R*)-3-methylpiperazin-1-yl]-3-(1*H-*pyrrolo[2,3-*b*]pyridin-4-yl)imidazo[1,2-*b*]pyridazine;
2. 6-(3,3-Dimethylpiperazin-1-yl)-2-methyl-3-(1*H-*pyrrolo[2,3-*b*]pyridin-4-yl)imidazo[1,2-*b*]pyridazine and the trihydrochloride thereof;
3.6-[(*cis*)-3,5-Dimethylpiperazin-1-yl]-2-methyl-3-(1*H*-pyrrolo[2,3-*b*]pyridin-4-yl)imidazo[1,2-*b*]pyridazine and the trihydrochloride thereof;
4. 6-(4-Isopropylpiperazin-1-yl)-2-methyl-3-(1*H-*pyrrolo[2,3-*b*]pyridin-4-yl)imidazo[1,2-*b*]pyridazine and the trihydrochloride thereof;
5. 2-Methyl-6-[(cis)-5-methylhexahydropyrrolo[3,4-*c*]pyrrol-2(1*H*)-yl]-3-(1*H*-pyrrolo[2,3-*b*]pyridin-4-yl)imidazo[1,2-*b*]pyridazine and the trihydrochloride thereof;
6. 2-(4-Fluorophenyl)-6-[(3*R*)-3-methylpiperazin-1-yl]-3-(1*H*-pyrrolo[2,3-*b*]pyridin-4-yl)imidazo[1,2-*b*]pyridazine;
7. {4-[2-(4-Fluorophenyl)-3-(1*H*-pyrrolo[2,3-*b*]pyridin-4-yl)imidazo[1,2-*b*]pyridazin-6-yl]-piperazin-2-yl}methanol;
8. 6-(3,3-Dimethylpiperazin-1-yl)-2-(4-fluorophenyl)-3-(1*H*-pyrrolo[2,3-*b*]pyridin-4-yl)imidazo[1,2-*b*]pyridazine;
9.6-(3,3-Dimethylpiperazin-1-yl)-2-(3-fluorophenyl)-8-methyl-3-(1*H*-pyrrolo[2,3-*b*]pyridin-4-yl)imidazo[1,2-*b*]pyridazine;
10. 6-(3,3-Dimethylpiperazin-1-yl)-2-(4-fluorophenyl)-8-methyl-3-(1*H*-pyrrolo[2,3-*b*]pyridin-4-yl)imidazo[1,2-*b*]pyridazine;
11. 6-[(*cis*)-3,5-Dimethylpiperazin-1-yl]-2-(4-fluorophenyl)-3-(1*H*-pyrrolo[2,3-*b*]pyridin-4-yl)imidazo[1,2-b]pyridazine;
12. 2-{4-[2-(3-Fluorophenyl)-8-methyl-3-(1*H-*pyrrolo[2,3-*b*]pyridin-4-yl)imidazo[1,2-*b*]pyridazin-6-yl]piperazin-1-yl}ethanol;
13. 2-{4-[2-(4-Fluorophenyl)-8-methyl-3-(1H-pyrrolo[2,3-*b*]pyridin-4-yl)imidazo[1,2-*b*]pyridazin-6-yl]piperazin-1-yl}ethanol;
14. 2-(4-Fluorophenyl)-6-(4-isopropylpiperazin-1-yl)-3-(1*H*-pyrrolo[2,3-*b*]pyridin-4-yl)imidazo[1,2-*b*]pyridazine;
15. 2-(4-Fluorophenyl)-6-(4-isopropylpiperazin-1-yl)-8-methyl-3-(1*H*-pyrrolo[2,3-*b*]pyridin-4-yl)imidazo[1,2-*b*]pyridazine;
16. (*R*)-1-{4-[2-(4-Fluorophenyl)-8-methyl-3-(1*H-*pyrrolo[2,3-*b*]pyridin-4-yl)imidazo[1,2-*b*]pyridazin-6-yl]piperazin-1-yl}propan-2-ol;
17. (*S*)-1-{4-[2-(4-Fluorophenyl)-8-methyl-3-(1H-pyrrolo[2,3-*b*]pyridin-4-yl)imidazo[1,2-*b*]pyridazin-6-yl]piperazin-1-yl}propan-2-ol;
18. 6-(6,9-Diazaspiro[4.5]dec-9-yl)-2-(4-fluorophenyl)-8-methyl-3-(1*H*-pyrrolo[2,3-*b*]pyridin-4-yl)imidazo[1,2-*b*]pyridazine;
19. 2-{4-[2-(4-Fluorophenyl)-3-(1*H*-pyrrolo[2,3-*b*]pyridin-4-yl)imidazo[1,2-*b*]pyridazin-6-yl]piperazin-1-yl}-2-methylpropan-1-ol;
20. 1-{4-[2-(4-Fluorophenyl)-3-(1*H*-pyrrolo[2,3-*b*]pyridin-4-yl)imidazo[1,2-b]pyridazin-6-yl]piperazin-1-yl}-2-methylpropan-2-ol;
21. 1-{4-[2-(3-Fluorophenyl)-8-methyl-3-(1H-pyrrolo[2,3-*b*]pyridin-4-yl)imidazo[1,2-*b*]pyridazin-6-yl]piperazin-1-yl}-2-methylpropan-2-ol;
22. 1-{4-[2-(4-Fluorophenyl)-8-methyl-3-(1*H-*pyrrolo[2,3-*b*]pyridin-4-yl)imidazo[1,2-*b*]pyridazin-6-yl]piperazin-1-yl}-2-methylpropan-2-ol;
23. 4-{4-[2-(4-Fluorophenyl)-3-(1*H*-pyrrolo[2,3-*b*]pyridin-4-yl)imidazo[1,2-*b*]pyridazin-6-yl]piperazin-1-yl}-2-methylbutan-2-ol;
24. (R)-2-(4-Fluorophenyl)-6-[3-phenylpiperazin-1-yl]-3-(1*H*-pyrrolo[2,3-*b*]pyridin-4-yl)-imidazo[1,2-*b*]pyridazine and the trihydrochloride thereof;
25. (*S*)-2-(4-Fluorophenyl)-6-[3-phenylpiperazin-1-yl]-3-(1*H*-pyrrolo[2,3-*b*]pyridin-4-yl)-imidazo[1,2-*b*]pyridazine and the trihydrochloride thereof;
26. 2-(4-Fluorophenyl)-8-methyl-6-[3-phenylpiperazin-1-yl]-3-(1*H*-pyrrolo[2,3-*b*]pyridin-4-yl)imidazo[1,2-*b*]pyridazine;
27. 6-(4-Benzylpiperazin-1-yl)-2-(4-fluorophenyl)-3-(1*H*-pyrrolo[2,3-*b*]pyridin-4-yl)-imidazo[1,2-*b*]pyridazine;
28. (*cis*)-2-(4-Fluorophenyl)-6-(5-methylhexahydropyrrolo[3,4-*c*]pyrrol-2(1*H*)-yl)-3-(1*H*-pyrrolo[2,3-*b*]pyridin-4-yl)imidazo[1,2-*b*]pyridazine;
29. (*cis*)-2-(4-Fluorophenyl)-8-methyl-6-(5-methylhexahydropyrrolo[3,4-*c*]pyrrol-2(1*H*)-yl)-3-(1*H*-pyrrolo[2,3-*b*]pyridin-4-yl)imidazo[1,2-*b*]pyridazine;
30. (*cis*)-2-{5-[2-(4-Fluorophenyl)-3-(1*H-*pyrrolo[2,3-*b*]pyridin-4-yl)imidazo[1,2-*b*]pyridazin-6-yl]hexahydropyrrolo[3,4-*c*]pyrrol-2(1*H*)-yl}ethanol;
31. (*cis*)-2-{5-[2-(4-Fluorophenyl)-8-methyl-3-(1*H*-pyrrolo[2,3-*b*]pyridin-4-yl)imidazo[1,2-*b*]pyridazin-6-yl]hexahydropyrrolo[3,4-*c*]pyrrol-2(1*H*)-yl}ethanol;
32. 2-(4-Fluorophenyl)-8-methyl-6-((*4aR*,*7aR*)-1-methyloctahydro-6*H*-pyrrolo[3,4-*b*]pyridin-6-yl)-3-(1*H*-pyrrolo[2,3-*b*]pyridin-4-yl)imidazo[1,2-*b*]pyridazine;
33. 2-(4-Fluorophenyl)-8-methyl-6-((*4aS*,*7aS*)-1-methyloctahydro-6*H*-pyrrolo[3,4-*b*]pyridin-6-yl)-3-(1*H*-pyrrolo[2,3-*b*]pyridin-4-yl)imidazo[1,2-*b*]pyridazine;
34. 2-(4-Fluorophenyl)-6-((1*S*,4*S*)-5-methyl-2,5-diazabicyclo[2.2.1]hept-2-yl)-3-(1*H*-pyrrolo[2,3-*b*]pyridin-4-yl)imidazo[1,2-*b*]pyridazine;
35. 9-[2-(4-Fluorophenyl)-3-(1*H*-pyrrolo[2,3-*b*]pyridin-4-yl)imidazo[1,2-*b*]pyridazin-6-yl]-2,9-diazaspiro[5.5]undecane;
36. 2-(4-Fluorophenyl)-6-(4-pyrrolidin-1-ylpiperidin-1-yl)-3-(1*H*-pyrrolo[2,3-*b*]pyridin-4-yl)imidazo[1,2-*b*]pyridazine.

13. Process for preparing a compound of general formula (I) according to Claim 1, **characterized in that** a compound of general formula (II): 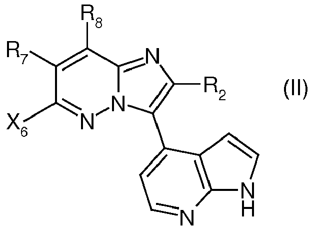 in which R₂, R₇ and R₈ are as defined according to Claim 1 and X₆ represents a leaving group, is reacted with an amine of general formula (III): 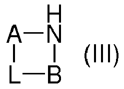 in which A, L and B are as defined according to Claim 1.

14. Process for preparing a compound of general formula (I) according to Claim 1, **characterized in that** a compound of general formula (V): 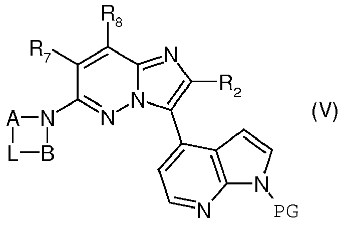 in which R₂, A, L, B, R₇ and R₈ are as defined according to Claim 1 and PG represents a benzene or toluenesulphonyl group, is deprotected using a base.

15. Medicament, **characterized in that** it comprises a compound of formula (I) according to any one of Claims 1 to 12, in the form of a base or an addition salt with a pharmaceutically acceptable acid.

16. Pharmaceutical composition, **characterized in that** it comprises a compound of formula (I) according to any one of Claims 1 to 12, in the form of a base or of an addition salt with a pharmaceutically acceptable acid, and also at least one pharmaceutically acceptable excipient.

17. Use of a compound of general formula (I) according to any one of Claims 1 to 12, for the preparation of a medicament intended for preventing or treating sleep disorders, circadian rhythm disorders, mood disorders, anxiety and depressives disorders, diseases associated with a dependence on abuse substances, diseases related to hyperphosphorylation of the tau protein, diseases caused or exacerbated by cell proliferation or inflammatory diseases.
